# EUROPEAN PATENT SPECIFICATION

(11) **EP 3 727 451 B1**
(45) Date of publication and mention of the grant of the patent: **10.05.2023**
(21) Application number: 18890486.6
(22) Date of filing: 21.12.2018
(51) Int. Cl.: A61K 45/06, C12N 1/06, C12N 9/14, A61K 38/47, A61P 31/04, A61Q 17/00, C11D 3/48

(54) **RECOMBINANT PSEUDOMONAS AERUGINOSA LYSINS**
REKOMBINANTE PSEUDOMONAS-AERUGINOSA-LYSINE
LYSINES DE PSEUDOMONAS AERUGINOSA RECOMBINÉES

(30) Priority: 22.12.2017 US 201762609969 P
(43) Date of publication of application: 28.10.2020
(73) Proprietor: The Rockefeller University, New York, NY 10021-6399 (US)
(72) Inventor: FISCHETTI, Vincent, Hempstead, NY 11552 (US); RAZ, Assaf, New York, NY 10021 (US); EULER, Chad, Forest Hills, NY 11375 (US)
(74) Representative: FRKelly
(86) International application number: PCT/US2018/067144
(87) International publication number: WO 2019/126670

(56) References cited:
- EP-A1- 3 813 796
- WO-A1-2015/070912
- WO-A1-2019/118632
- WO-A2-2017/049233
- WO-A2-2017/049242
- KR-A- 20170 061 553
- US-A1- 2015 198 595
- RAZ ASSAF ET AL: "Isolation of Phage Lysins That Effectively Kill Pseudomonas aeruginosa in Mouse Models of Lung and Skin Infection", ANTIMICROBIAL AGENTS AND CHEMOTHERAPY, vol. 63, no. 7, 1 July 2019 (2019-07-01), XP055810428, US ISSN: 0066-4804, DOI: 10.1128/AAC.00024-19 Retrieved from the Internet: URL:https://www.ncbi.nlm.nih.gov/pmc/artic les/PMC6591642/pdf/AAC.00024-19.pdf>
- Anonymous ET AL: "the lysin meeting", , 1 January 2018 (2018-01-01), XP055810437, Retrieved from the Internet: URL:https://www.thelysinmeeting.com/wp-con tent/uploads/2018/10/AGENDA-DRAFT-Lysin-Me eting-10.19.2018.pdf [retrieved on 2021-06-04]
- ROLF LOOD ET AL: "Novel Phage Lysin Capable of Killing the Multidrug-Resistant Gram-Negative Bacterium Acinetobacter baumannii in a Mouse Bacteremia Model", ANTIMICROBIAL AGENTS AND CHEMOTHERAPY, vol. 59, no. 4, 11 April 2015 (2015-04-11) , pages 1983-1991, XP055330866, US ISSN: 0066-4804, DOI: 10.1128/AAC.04641-14
- HANG YANG ET AL: "Antibacterial Activity of a Novel Peptide-Modified Lysin Against Acinetobacter baumannii and Pseudomonas aeruginosa", FRONTIERS IN MICROBIOLOGY, vol. 6, no. 1471, 22 December 2015 (2015-12-22), pages 1-9, XP055657170, DOI: 10.3389/fmicb.2015.01471
- MYA THANDAR ET AL: "Novel Engineered Peptides of a Phage Lysin as Effective Antimicrobials against Multidrug-Resistant Acinetobacter baumannii", ANTIMICROBIAL AGENTS AND CHEMOTHERAPY, vol. 60, no. 5, 8 February 2016 (2016-02-08), pages 2671-2679, XP055650498, US ISSN: 0066-4804, DOI: 10.1128/AAC.02972-15
- GUO MINGQUAN ET AL: "A Novel Antimicrobial Endolysin, LysPA26, against Pseudomonas aeruginosa", FRONTIERS IN MICROBIOLOGY, vol. 8, 1 January 2017 (2017-01-01), page 293, XP055810486, DOI: 10.3389/fmicb.2017.00293 Retrieved from the Internet: URL:https://www.ncbi.nlm.nih.gov/pmc/artic les/PMC5326749/pdf/fmicb-08-00293.pdf>

## Description

### FIELD OF THE DISCLOSURE

The disclosure relates to bacteriophage lysins and derivatives that kill bacterial cells on contact.

### BACKGROUND OF THE DISCLOSURE

*Pseudomonas aeruginosa* is an environmental Gram-negative bacterium that exhibits extensive metabolic adaptability, enabling it to thrive in an extraordinary range of niches (Silby, Winstanley et al. 2011). It is a highly successful opportunistic pathogen, causing a wide range of acute and chronic infections (Winstanley, O'Brien et al. 2016), and is a leading cause of nosocomial infections worldwide (Spencer 1996) (Koulenti, Lisboa et al. 2009). Since the early 1990s there has been an increasing rate of *P. aeruginosa* involvement in serious infections with high mortality rates (Livermore 2002). *P. aeruginosa* predominantly infects patients with compromised host defenses, such as in the case of severe burns, cystic fibrosis (CF), and neutropenia (Lyczak, Cannon et al. 2000). Additionally, many infections initiate from environmental sources (for example those of CF patients), and are thus difficult to control (Kidd, Ramsay et al. 2013). *P. aeruginosa* is inherently resistant to many antimicrobial classes due to the limited permeability of its outer membrane (Nicas and Hancock 1983) (Hancock 1998). It is also capable of acquiring antimicrobial resistance to all relevant antibiotics through mutations or the acquisition of new genetic material, severely limiting the treatment options available (El Solh and Alhajhusain 2009). For multidrug-resistant (MDR) *P. aeruginosa,* polymixins represent antibiotics of last resort despite excessive toxicity (Evans, Feola et al. 1999) (Livermore 2002) (Falagas, Rizos et al. 2005) (Li, Nation et al. 2006). However, resistance to polymyxins is also increasing, resulting in truly pan-resistant strains for which no effective antibiotics are available (Falagas, Bliziotis et al. 2005) (Sonnevend, Ghazawi et al. 2017) (Weterings, Zhou et al. 2015). Similar trends in antibiotic resistance have also been observed in other Gram-negative bacteria including, *Acinetobacter* spp. and various Enterobacteriaceae. Bacteria producing extended-spectrum beta-lactamases (ESBLs) have become a major public health concern globally (Pitout and Laupland 2008). Resistance to broad-spectrum antibiotics such as third-generation cephalosporins (e.g., ceftazidime and ceftriaxone) in *Escherichia coli* and *Klebsiella pneumoniae* is widespread. A concomitant increase in the use of carbapenems has led to a rapid increase in the rate of carbapenem-resistant Enterobacteriaceae (CRE) in the nosocomial setting, with a high mortality rate (Chang, Hsu et al. 2011) (Cornaglia, Giamarellou et al. 2011) (Falagas, Tansarli et al. 2014). There is therefore a clear and urgent need for new therapeutics for MDR Gram-negative bacteria, as well as other bacteria. The present disclosure is pertinent to this need.
WO 2015/070912A1 discloses a bacteriophage lysin having the ability to degrade the peptidoglycan of Gram-negative bacteria, in particular of *Pseudomonas* bacteria.
Hang Yang et al: "Antibacterial Activity of a Novel Peptide-Modified Lysin Against Acinetobacter baumannii and Pseudomonas aeruginosa", Frontiers in Microbiology, vol. 6, no. 1471, 22 December 2015, pages 1-9, DOI: 10.3389/fmicb.2015.01471
indicates that modifying a natural lysin with an antimicrobial peptide has been found able to break the barriers, and to kill Gram-negative pathogens.
KR 2017 0061553 A discloses bacteriophage lysins against *P*. *Aeruginosa.*
WO 2017/049233 A2 and WO 2017/049242 A2 are directed to treating a subject afflicted with a bacterial infection by co-administering an antibiotic and a lysin polypeptide. The lysins have antibacterial activity against infections caused by *P. aeruginosa* and *S. aureus.*

### SUMMARY

The present invention provides a pharmaceutical composition comprising at least one isolated and/or recombinant lysin polypeptide that is effective to kill bacteria, wherein the lysin polypeptide comprises an amino acid sequence that is 100% identical to an amino acid sequence of a lysin polypeptide of PlyPa91 (SEQ ID NO: 13).

In embodiments, the lysin polypeptide is a recombinant polypeptide. In embodiments, the lysin polypeptide comprises amino acids encoded by an expression vector used to produce the polypeptide, and/or comprise an amino acid sequence of an anti-microbial peptide. In embodiments, the lysin used in compositions and methods isPlyPa91 (SEQ ID NO:13).

In another aspect, the disclosure comprises a method of killing bacteria on or in an individual by contacting bacteria with an effective amount of a pharmaceutical composition comprising the lysin described herein, wherein the bacteria is from *Enterobacteriaceae;* or is from the genera *Enterobacter, Pseudomonas* or *Klebsiella,* or wherein the bacteria is *A. baumannii, E. coli, S. sonnei, S. flexneri, C. freundii, K. pneumoniae, E. cloacae, E. aerogenenes* or *P. aeruginosa.* In general, the bacteria that are killed are Gram-negative bacteria. In embodiments, the Gram-negative bacteria are in an infection of skin, a wound, and/or mucosa of the individual. In embodiments, the Gram-negative bacteria are *Enterobacteriaceae.* In embodiments, the Gram-negative bacteria are *Enterobacter,* including but not necessarily limited to *Enterobacter aerogenes* or *Enterobacter cloacae.* In embodiments, the Gram-negative bacteria are *Pseudomonas,* including but not necessarily limited to *Pseudomonas aeruginosa.* In embodiments, the Gram-negative bacteria are *Klebsiella,* including but not necessarily limited to *Klebsiella pneumonia.* In embodiments, the Gram-negative bacteria are *Pseudomonas aeruginosa* and/or *Klebsiella pneumonia* and are present in a lung infection in the individual, and/or have colonized lungs of the individual.

In another aspect, the disclosure provides an expression vector encoding the recombinant lysin PlyPa91. In embodiments, the encoded recombinant polypeptide optionally comprises at least one of: a purification tag, or an amino acid sequence encoded by the expression vector from codons of one or more restriction enzyme recognition sites, or a sequence encoding a protease recognition sequence.

Cells comprising expression vectors encoding PlyPa91 are included in the disclosure. Such cells can be used, for example, to make the lysin. In one approach, the disclosure thus provides a method comprising such cells, separating the recombinant lysin polypeptide PlyPa91 from the cells, and optionally purifying the recombinant lysin polypeptides for use in a pharmaceutical formulation, and optionally removing a purification tag using a sequence-dependent protease that recognizes a protease cleavage site that has been incorporated into the polypeptide.

In another aspect, the disclosure comprises an article of manufacture comprising a pharmaceutical composition comprising any lysin or combination thereof. The article further comprises printed material providing an indication that the composition is for use in killing bacteria.

In another aspect, the disclosure comprises a method, the method comprising contacting a sample with a detectably labeled lysin, PlyPa91, and detecting a signal from the detectable label on the lysin to thereby determine the presence of bacteria in the sample. This approach may be followed by treatment using the lysin without the label that was used in the detection. Thus, the method may further comprise determining the type of bacteria in the sample based on the amino acid sequence of the detectably labeled lysin, and treating the individual from which the sample was obtained using the lysin.

In another aspect, the disclosure comprises a non-claimed method of detection and/or identification of bacteria wherein the lysin is labeled and used to for detection or identification of the bacteria by methods that include but are not limited to ELISA, Flow cytometry, or microscopy. Alternatively, labeled or unmodified lysin could be used to lyse target organisms, and cell lysis could be monitored using methods such as, but not limited to, ATP release, thereby confirming the presence of organisms susceptible to the lysin in the target sample. Thus, the method may further comprise determining the type of bacteria in the sample based on the binding of the lysin or the susceptibility to lysin, and optionally treating the individual from which the sample was obtained using the lysin.

### BRIEF DESCRIPTION OF THE DRAWINGS

In bar graphs of the figures, unless otherwise indicated, where a key is provided, the key represents, from top to bottom, the bars on the X-axis of the graph, from left to right for each result shown.

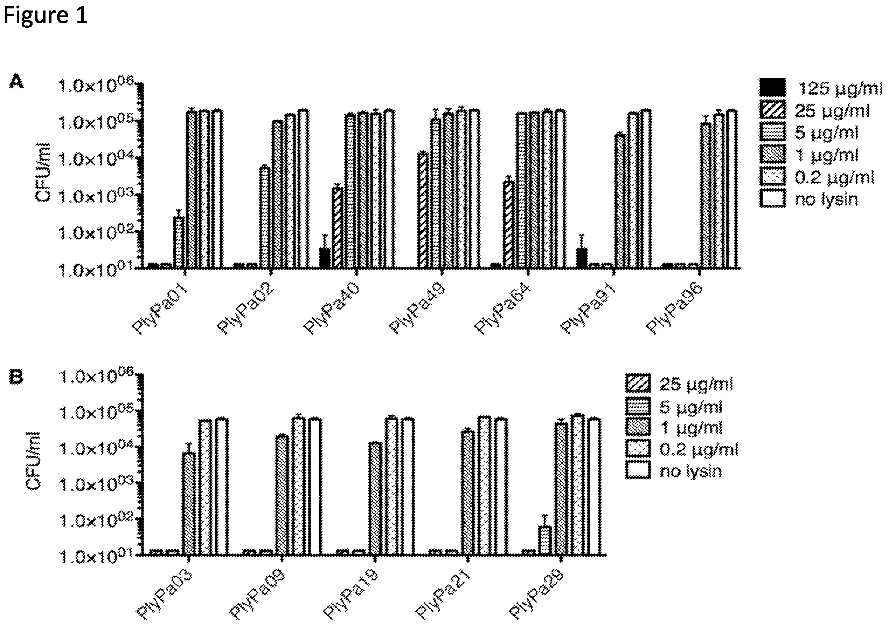

**Fig. 1** - **Killing activity of purified lysins against *P. aeruginosa* PA01.** Purified and 3C-cleaved lysins were diluted to various concentrations and incubated with log-phase *P*. *aeruginosa* PA01 for 1 h at 37°C in 30 mM HEPES pH 7.4. CFU/ml values were established by serial dilution and plating. Experiments were conducted in duplicates. **(A)** Initial lysins. **(B)** Additional homologues of PlyPa02.

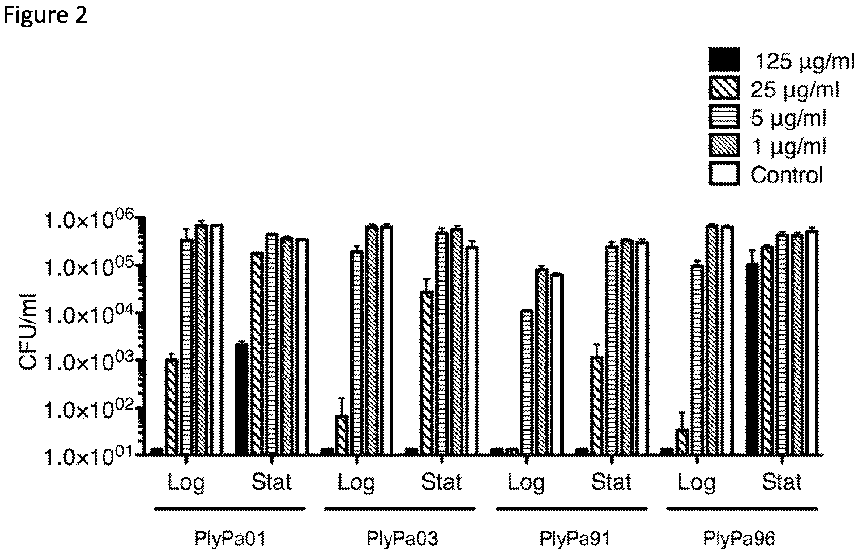

**Fig. 2** - **Activity of the lysins against log-phase and stationary** ***P. aeruginosa.** P. aeruginosa* were grown overnight (Stat), diluted 1:100 and grown to log-phase (Log). Bacteria were washed, and incubated with lysins at the indicated concentrations in 30 mM HEPES buffer pH 7.4 for 1 h at 37°C. Viable bacteria were quantified by serial dilution and plating. Experiments were done in duplicates.

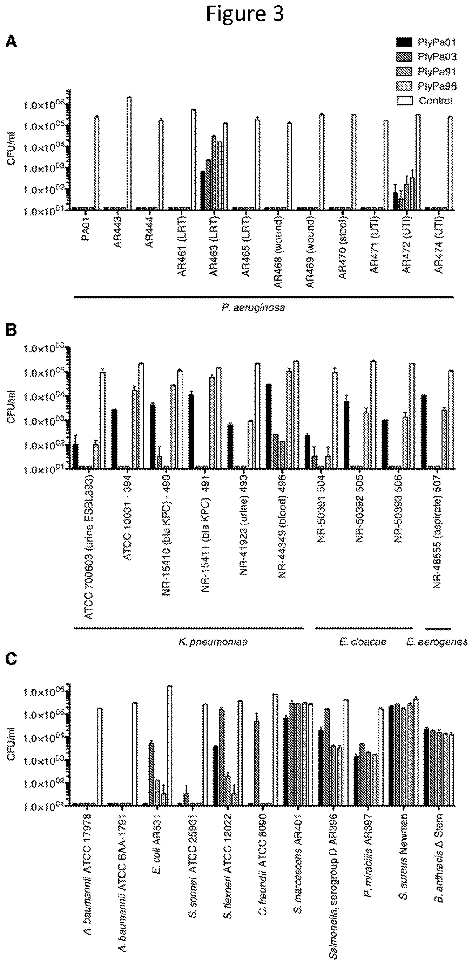

**Fig. 3** - Activity of PlyPa01, PlyPa03, PlyPa91 and PlyPa96 against various bacteria. Various isolates of *P. aeruginosa* (A), *Klebsiella andEnterobacter* (B), and other Gram-negative and Gram-positive bacteria (C), were incubated with 100 µg/ml lysins in 30 mM HEPES buffer pH 7.4 for 1 h at 37°C. Viable bacteria were enumerated by serial dilution and plating. PlyPa01, PlyPa03, PlyPa91, PlyPa96, and control shown left to right in each group.

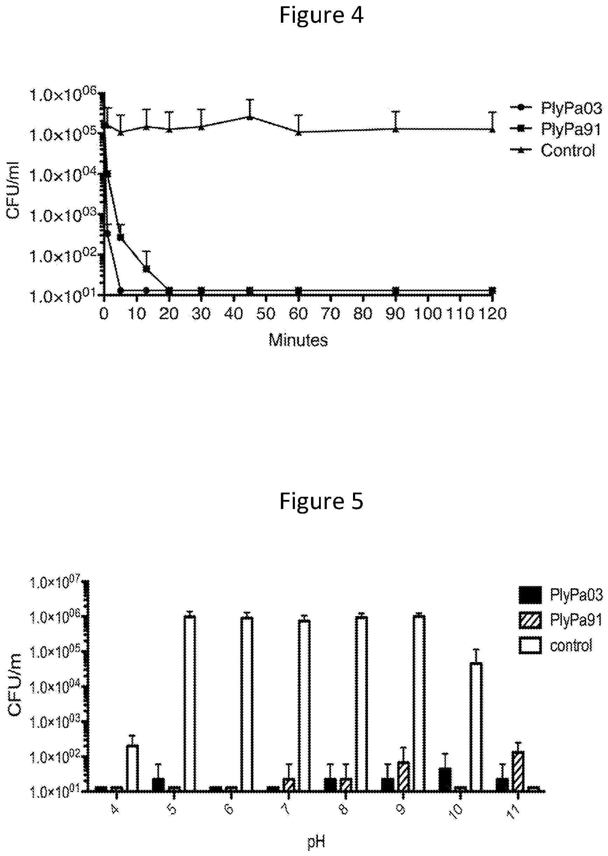

**Fig. 4** - **Time kill curve** - Log-phase *P. aeruginosa* PA01 cells were incubated for varying lengths of time at 37°C with 100 µg/ml lysin in 30mM HEPES buffer. Surviving bacteria were enumerated by serial dilution and plating; experiments were done in triplicates.
**Fig. 5** - **Effect of pH on the activity of PlyPa03 and PlyPa91.** Log-phase *P*. *aeruginosa* PA01 cells were incubated for 1 h at 37°C with 100 µg/ml lysin in 25 mM of the following buffers: pH 4.0 and 5.0 - acetate buffer; pH 6.0 - MES buffer; pH 7.0 and 8.0 - HEPES buffer; pH 9.0 - CHES buffer; pH 10.0 and 11.0 - CAPS buffer. Surviving bacterial CFU/ml are presented; experiments were performed in triplicates.

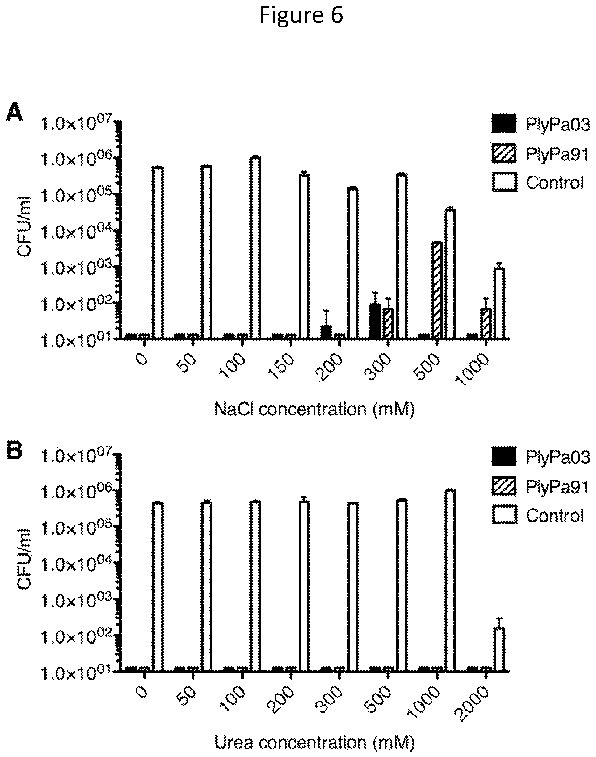

**Fig. 6** **- Effect of NaCl and urea on the activity of PlyPa03 and PlyPa91.** Log-phase *P*. *aeruginosa* PA01 cells were incubated with 100 µg/ml PlyPa03, or PlyPa91 for 1 h at 37°C in 30 mM HEPES pH 7.4 and various concentrations of NaCl **(A)** or urea **(B).** Surviving bacterial CFU/ml are presented; experiments were performed in triplicates.

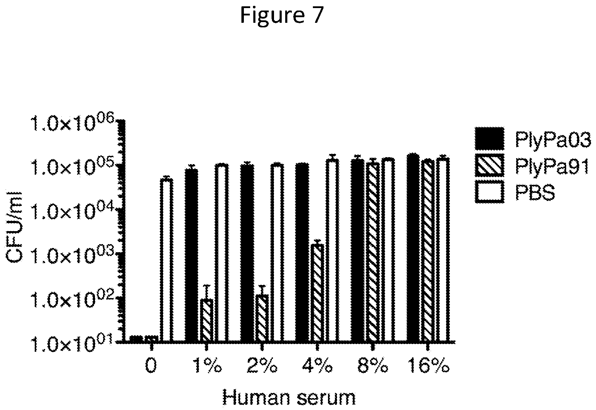

**Fig. 7** **- Activity of PlyPa03 and PlyPa91 in the presence of human serum.** *P*. *aeruginosa* PA01 cells were incubated for 1 h at 37°C with 100 µg/ml of the lysins in the presence of the indicated concentration of Serum. Viable bacterial CFU are presented. Experiments were done in triplicates.

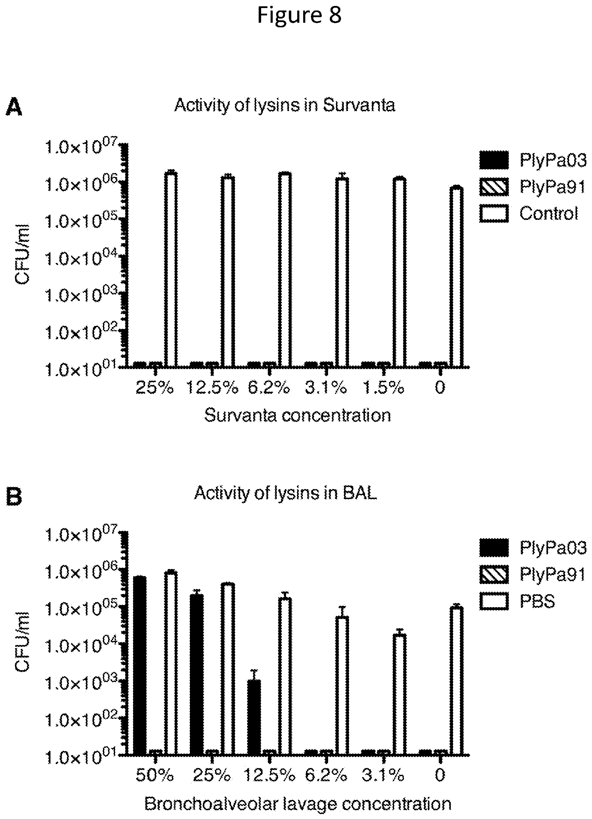

**Fig. 8** **- PlyPa03 and PlyPa91 are active in Survanta and Bronchoalveolar lavage (BAL)** Log-phase *P. aeruginosa* PA01 cells were incubated for 1 h at 37°C with 100 µg/ml of PlyPa03, PlyPa91, or buffer control, in the presence of the indicated concentration of the compound sold under the tradename Survanta (generic for beractan) (A) or freshly isolated bronchoalveolar lavage (B). Viable bacterial CFU were determined by serial dilution and plating. Experiments were done in triplicate.

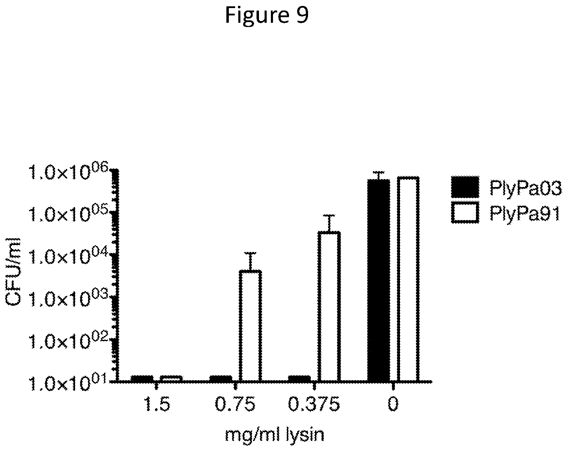

**Fig. 9** - **Elimination *of P. aeruginosa* biofilm by PlyPa03 and PlyPa91.** *P*. *aeruginosa* PA01 biofilm was established using the MBEC Biofilm Inoculator 96-well plate system. Biofilms were grown for 24 h on the 96-peg lid, washed twice, and treated with different concentrations of PlyPa03, PlyPa91, of buffer control for 2 h at 37°C. The pegs were washed, and surviving bacteria were recovered by sonication in 200 µl/well PBS. Quantification of surviving bacteria was done by serial dilution and plating. Experiments were done in triplicates and repeated twice.

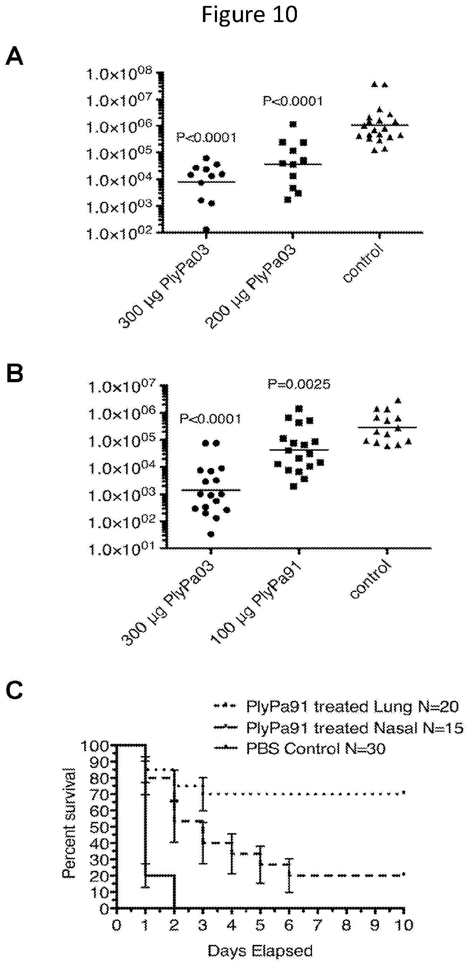

**Fig. 10** **- Lysins protects mice in skin and lung infection models. A and B.** A skin area on the backs of CD1 female mice was shaven and tape-stripped, and then infected with 10 µl Log-phase *P. aeruginosa* at 5 × 10⁶ CFU/ml. After 20 hours, the mice were treated with PlyPa03, PlyPa91, or buffer control, and were euthanized 3 hours later. The infected skin was immediately excised and homogenized in PBS, and the resulting liquid was serially diluted and plated for CFU quantification. Geometric mean of the values is presented (**A** and **B** represent two separate experiments). **C.** Lungs of female C57BL/6 mice were infected by intranasal application of 2 × 50 µl of 10⁸ CFU/ml log-phase *P. aeruginosa* PA01 by intranasal installation. At three and six hours post infection mice were treated with 50 µl of 1.8 mg/ml PlyPa91 or PBS by two intranasal installations (Nasal) or by one intranasal and one intratracheal installation (Lung); PBS controls from the two treatment regiments were combined in a single group. 10 day survival was analyzed using Kaplan-Meier survival curves with standard errors, 95% confidence intervals, and significance levels (log rank/Mantel-Cox test).

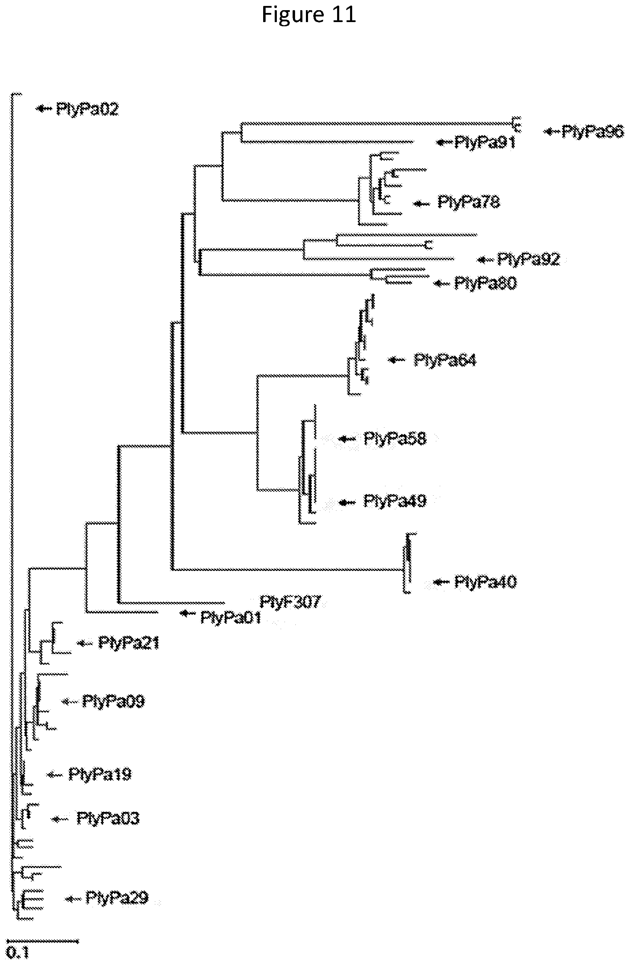

**Fig. 11** **- Phylogenetic tree of *P. aeruginosa* phage lysins with homology to PlyF307** Phage lysins were identified through homology search of the NCBI database using PlyF307 as query (black square). Sequences were analyzed using Lasergene MegAlign Pro with the MUSCLE algorithm, producing a phylogenetic tree. Lysins chosen for the initial screen are denoted with black arrows. Lysins chosen for the second step are denoted with arrows. Only lysins described in this disclosure are indicated on the tree.

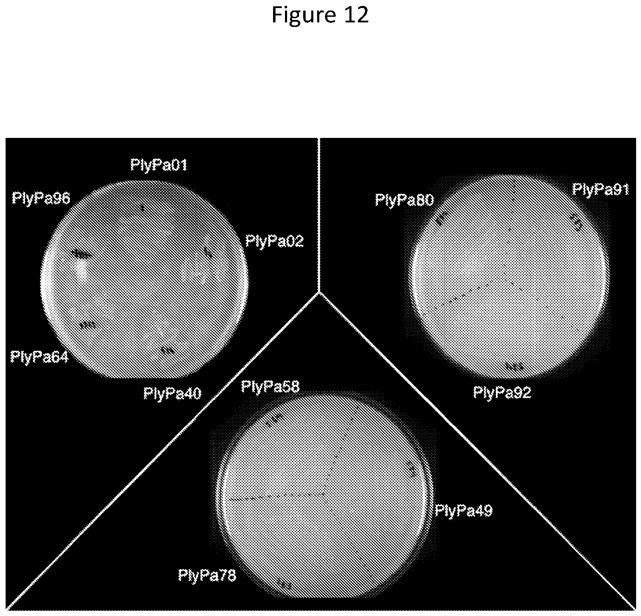

**Fig. 12** **- Evaluation of lysin peptidoglycan hydrolase activity using the plate overlay method.** *E. coli* strains containing lysin genes in pAR553 were grown on a plate containing 0.2% arabinose to induce lysin expression. Cells were permeabilized with chloroform vapor and overlayed with soft agar containing autoclaved *P. aeruginosa* cells. Enzymatic activity was evaluated by the appearance of clearing zones.

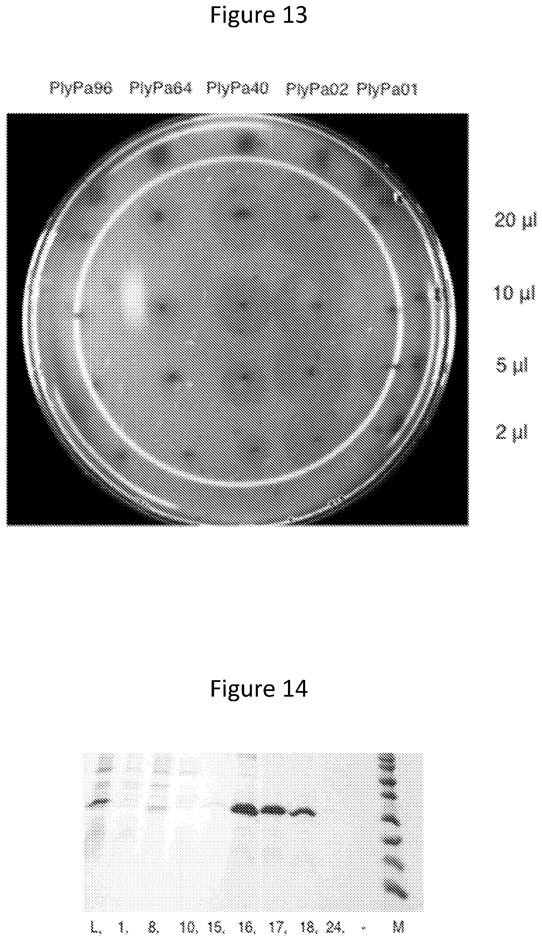

**Fig. 13** **- Evaluation of lysin peptidoglycan hydrolase activity in crude lysate.** Induced crude lysates of *E. coli* strains harboring the lysin genes in pAR553 were spotted in different amounts on a plate containing soft agar with autoclaved *P. aeruginosa.* Enzymatic activity was evaluated by the appearance of clearing zones.
**Fig. 14** **- purification of PlyPa02.** A PlyPa02 fused to a 3C-cleavable hexahistidine tag was purified from an induced *E. coli* lysate by a single step metal affinity chromatography: L - Induced lysate; fractions 1-5 - load; fractions 6-15 - wash steps; fractions 16-18 - collected elution; fractions 23-29 - column regeneration. The image shows a Coommassie stain of a 15% SDS-PAGE containing select fractions.

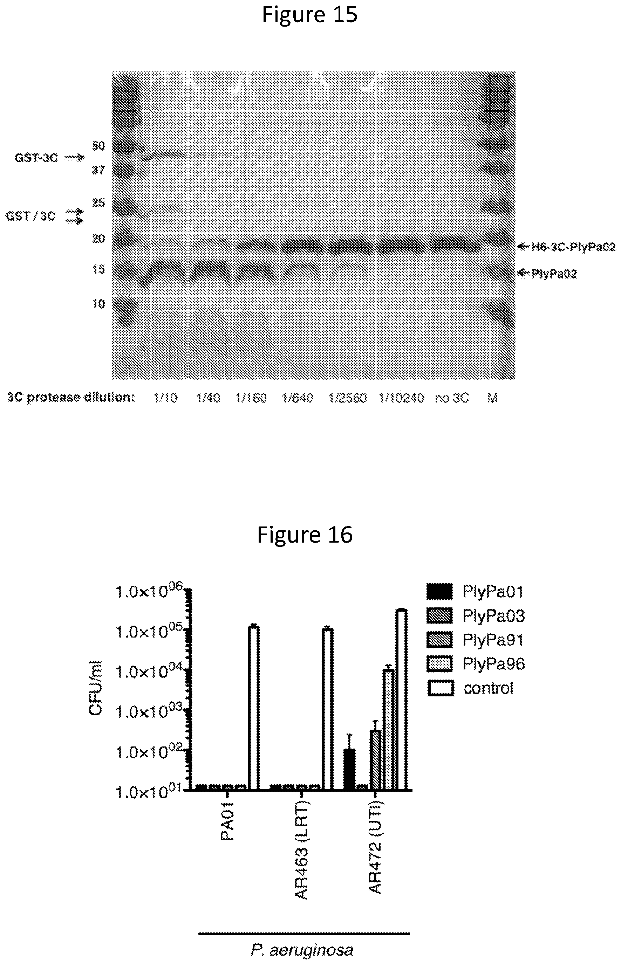

**Fig. 15** **- Cleavage of PlyPa02 with various doses of 3C protease.** Reaction mixtures with a total volume of 20 µl were prepared by combining 10 µg of PlyPa02, 2 µl of 4-fold serially diluted 3C protease and the following buffer composition: 150 mM NaCl; 50 mM tris; 10 mM EDTA; and 1 mM DTT, pH 7.6. Reactions were incubated at 4°C for 16 h, samples were loaded on 15% SDS-PAGE, and the gel stained with Coomassie blue.
**Fig. 16** **- Activity of lysins against *P. aeruginosa* strains at 250 µg/ml.** *P. aeruginosa* strains PA01, AR463, and AR463 were incubated with 250 µg/ml of the lysins in 30 mM HEPES buffer pH 7.4 for 1 h at 37°C. Viable bacteria were enumerated by serial dilution and plating.

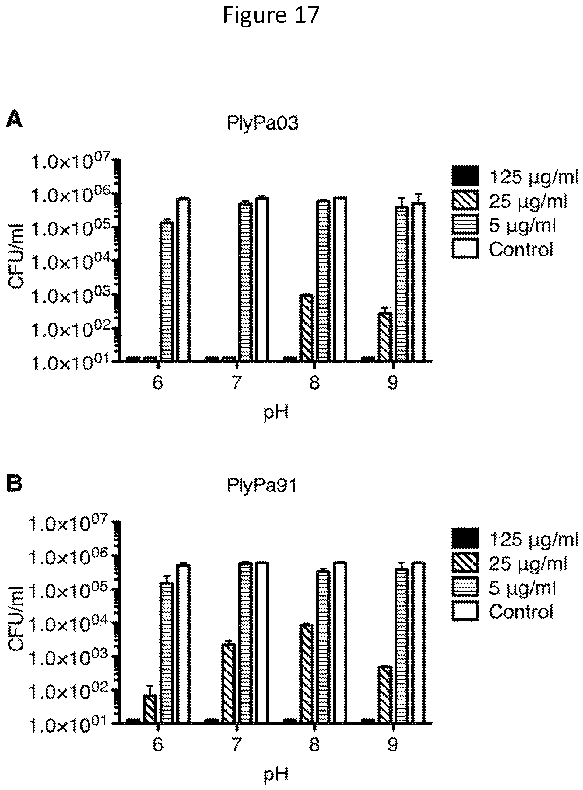

**Fig. 17** **- Effect of pH on the activity of PlyPa03 and PlyPa91.** Log-phase *P*. *aeruginosa* PA01 cells were incubated for 1 h at 37°C with various lysin concentrations in 25 mM of the following buffers: pH 6.0 - MES buffer; pH 7.0 and 8.0 - HEPES buffer; pH 9.0 - CHES buffer. Surviving bacterial CFU/ml are presented; experiments were performed in triplicates.

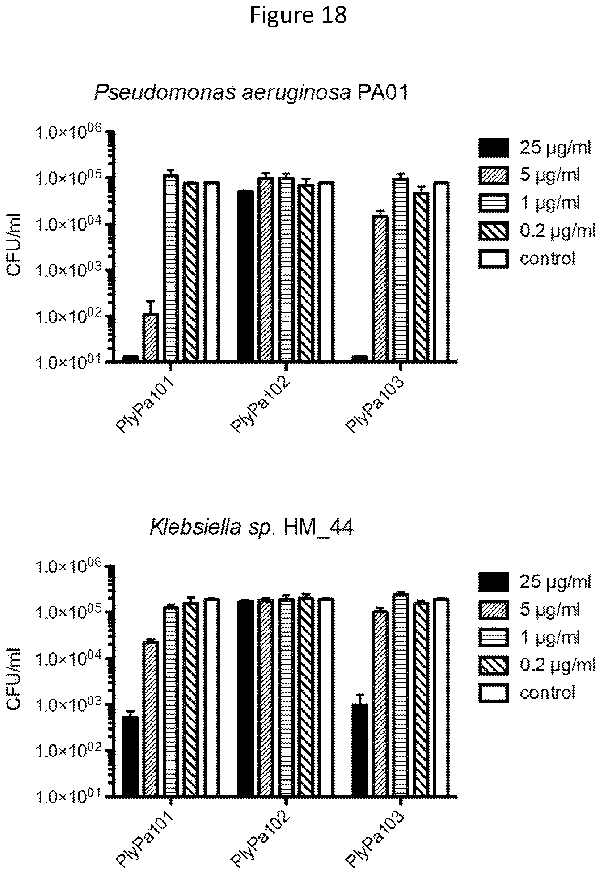

**Fig. 18****.** Log phase *P. aeruginosa* PA01 or *Klebsiella* spp. HM_44 were incubated with varying concentrations of PlyPa101, PlyPa102, or PlyPa103 in 30mM HEPES pH 7.4 for 1 h at 37C. surviving bacteria were evaluated by serial dilutions and plating.

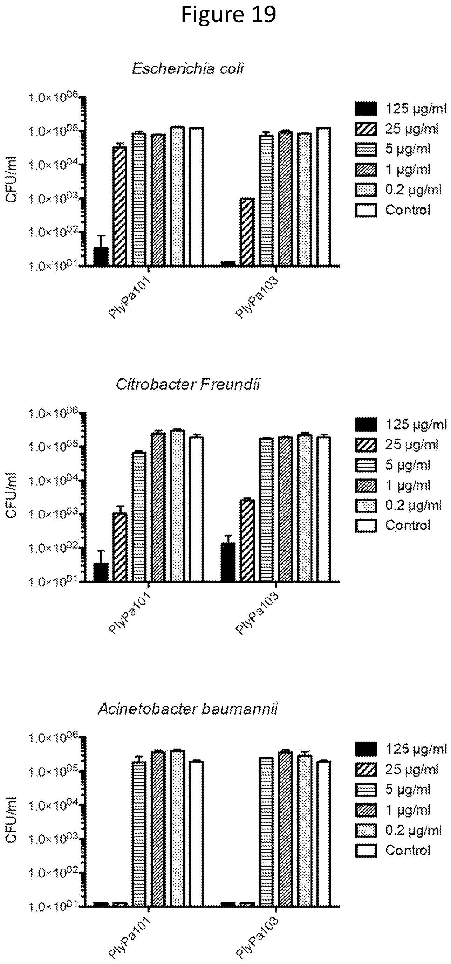

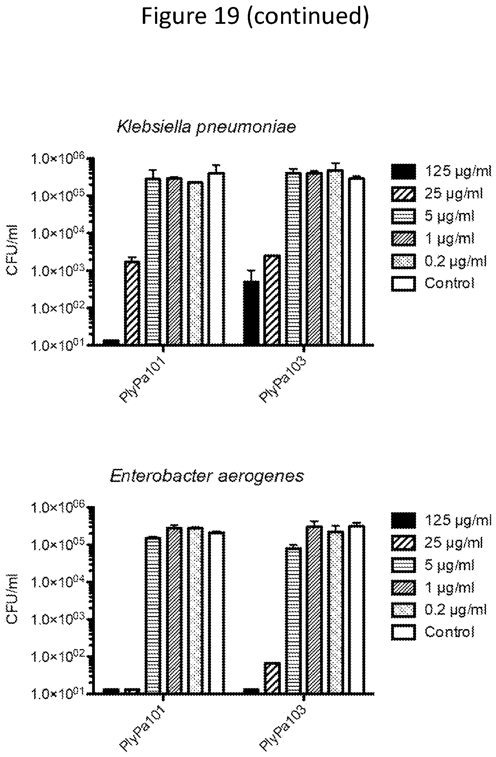

**Fig. 19****.** Log phase *E*. *coli, K. pneumoniae, C. freundii, E. aerogenes, and A. baumannii* were incubated with varying concentrations of Ply Pal 0 1, or PlyPa103 in 30mM HEPES pH 7.4 for 1 h at 37C. surviving bacteria were evaluated by serial dilutions and plating.

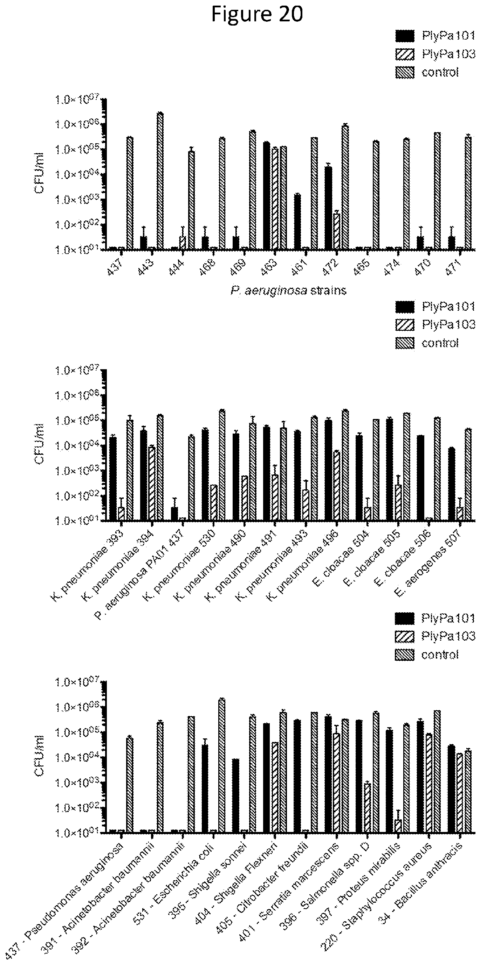

**Fig. 20****. Activity of PlyPa101 and PlyPa103 against various clinical isolates.** Log phase bacteria were incubated with 100 ug/ml of PlyPa101, or PlyPa103 in 30mM HEPES pH 7.4 for 1 h at 37C. surviving bacteria were evaluated by serial dilutions and plating.

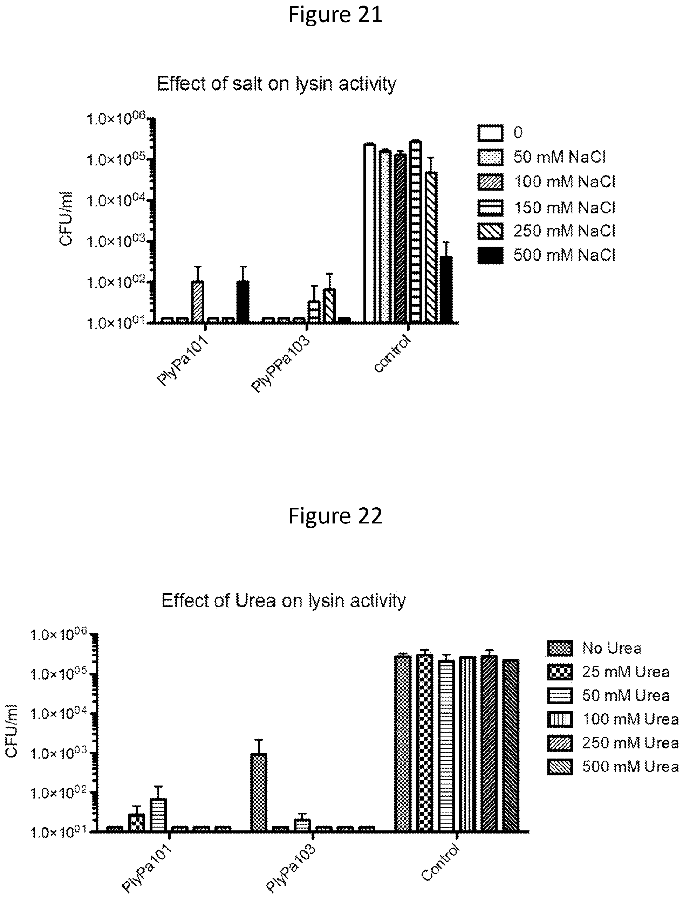

**Fig. 21****. Effect of salt on the activity of PlyPa101 and PlyPa103.** Log phase *P*. *aeruginosa* PA01 were incubated with 100 ug/ml of PlyPa101, or PlyPa103 in 30mM HEPES pH 7.4 and varying concentrations of NaCl, for 1 h at 37C. Surviving bacteria were evaluated by serial dilutions and plating.
**Fig. 22****. Effect of Urea on the activity of PlyPa101 and PlyPa103.** Log phase *P*. *aeruginosa* PA01 were incubated with 100 ug/ml of PlyPa101, or PlyPa103 in 30mM HEPES pH 7.4 and varying concentrations of Urea, for 1 h at 37C. Surviving bacteria were evaluated by serial dilutions and plating.

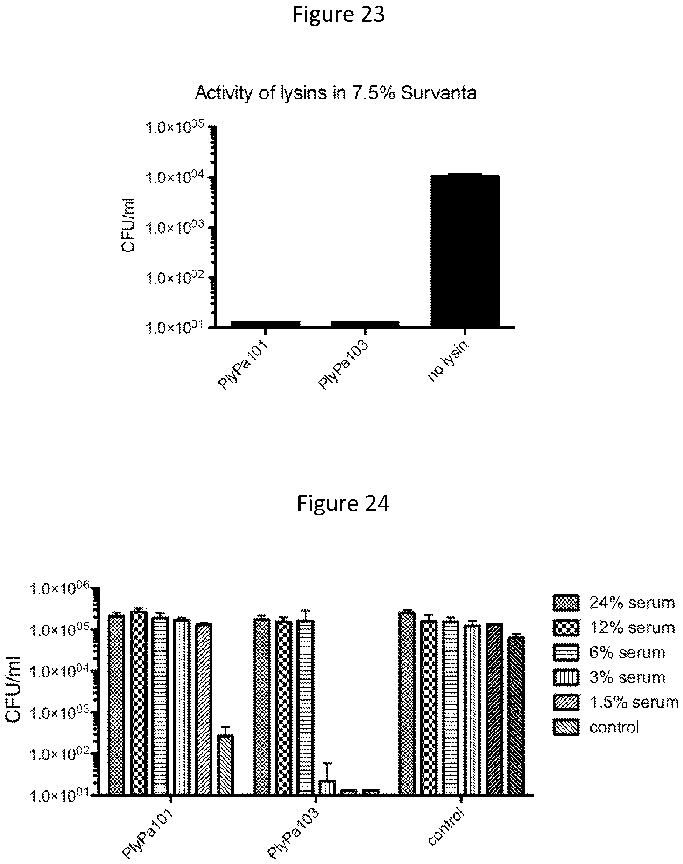

**Fig. 23****. Effect of Survanta on the activity of PlyPa101 and PlyPa103.** Log phase *P. aeruginosa* PA01 were incubated with 100 ug/ml of PlyPa101, or PlyPa103 in 30mM HEPES pH 7.4 and 7.5% Survanta, for 1 h at 37C. surviving bacteria were evaluated by serial dilutions and plating.
**Fig. 24****. Effect of human serum on the activity of PlyPa101 and PlyPa103.** Log phase *P. aeruginosa* PA01 were incubated with 100 ug/ml of PlyPa101, or PlyPa103 in 30mM HEPES pH 7.4 and varying concentrations of human serum (AB blood type pooled serum), for 1 h at 37C. Surviving bacteria were evaluated by serial dilutions and plating.

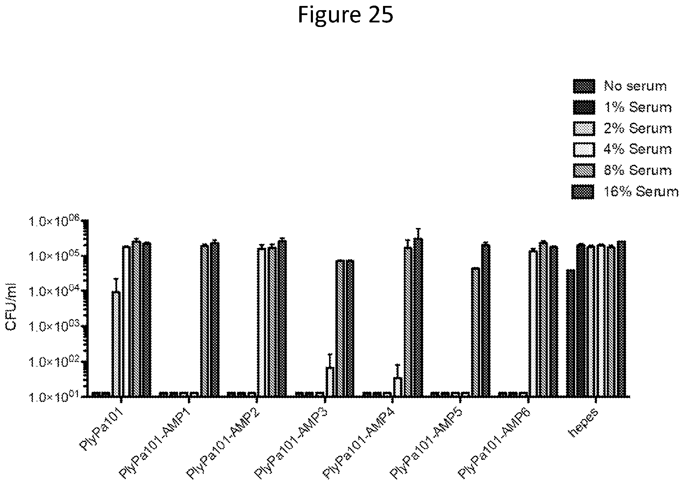

**Fig. 25****. Effect of fusion of antimicrobial peptides to PlyPa101 on activity in human serum.** Log phase *P. aeruginosa* PA01 were incubated with 100 ug/ml of PlyPa101, and PlyPa101 AMP fusion proteins, in 30mM HEPES pH 7.4 and varying concentrations of human serum (AB blood type pooled serum), for 1 h at 37C. Surviving bacteria were evaluated by serial dilutions and plating. AMP1=LL37, AMP2=LALF, AMP3=RI-18, AMP4=WLBU, AMP5=RP-1, AMP6=pexiganan.

### DETAILED DESCRIPTION

The present disclosure provides compositions comprising lysins, and methods of using the lysins to kill bacteria, as further described herein. The present invention relates to the lysin PlyPa91, but other lysins are disclosed herein as comparative examples.

Lysins are peptidoglycan hydrolases produced by bacteriophages to release progeny phage from an infected bacterial host. At the end of the phage replicative cycle, lysins gain access to the peptidoglycan through a pore, formed in the inner cell membrane by another phage product, the holin, and the resulting peptidoglycan hydrolysis leads to hypotonic rupture of the cell wall and release of progeny phages (Young 2014). Lysins can be endo-β-N-acetylglucosaminidases or N-acetyl-muramidases (lysozymes), which act on the sugar moiety, endopeptidases which act on the peptide backbone or cross bridge, or more commonly, an N-acetylmuramoyl-L-alanine amidase (or amidase), which hydrolyzes the amide bond connecting the sugar and peptide moieties. Significantly, exogenously added lysin can lyse the cell wall of healthy, uninfected Gram-positive bacteria, producing a phenomenon known as "lysis from without" (Schuch, Nelson et al. 2002) (Fischetti 2010) (Loeffler, Nelson et al. 2001) (Gilmer, Schmitz et al. 2013). Gram-negative bacteria on the other hand, have proven highly resistant to exogenously added lysins due to their protective outer membrane. Thus, the use of lysins in combination with membrane destabilizing factors is typically required for activity (Diez-Martinez, de Paz et al. 2013) (Walmagh, Briers et al. 2012). In a series of studies, Ibrahim et al. demonstrated that a cell wall hydrolase could become active against Gram-negative bacteria by adding a hydrophobic tail to the molecule (Ibrahim, Yamada et al. 1994) (Arima, Ibrahim et al. 1997). Additionally, a small fraction of natural lysins display activity against Gram-negative bacteria, and this activity is attributed to the presence of one or more amphipathic helixes in the molecule, responsible for membrane permeabilization (Morita, Tanji et al. 2001) (Orito, Morita et al. 2004) (Lai, Lin et al. 2011). One lysin, referred to as PlyF307, displayed significant killing activity of *A*. *baumannii,* and contained a positively charged C-terminal tail, required for killing activity (Lood, Winer et al. 2015).

The present disclosure provides in one aspect a phylogenetic analysis of the sequenced genomes of *P. aeruginosa* for phage lysins with homology to PlyF307, and further provides additional lysins, and modifications of the lysins. In this regard, Table 1 describes the amino acid sequence encoding *Pseudomonas aeruginosa* lysins that are encompassed by this disclosure. The amino acids in this Table do not include cleavable tags.

For 16 lysins described in Table 1, from which two lysins (PlyPa03 and PlyPa91) displayed substantial activity against a range of *Pseudomonas, Klebsiella, Enterobacter,* and other Gram-negative bacterial strains. Thus, only select lysins are suitable for killing bacteria. It is not apparent which lysins could exhibit this feature from sequence analysis. In this regard, these enzymes had robust activity in a wide pH range, and high salt and urea concentrations. The enzymes were active in the presence of the pulmonary surfactant Survanta, and were protective in murine models of *Pseudomonas* infection. Thus, any lysin described herein can be characterized based on its effect on bacteria, relative to the effect any other lysin(s) described herein.

For additional lysins in Table 1, as described in Example 2, an approach that is different from the phylogenetic analysis was used. Specifically, bacteriophage lysins were isolated from native bacteriophages. We amplified the genes encoding the lysins of phages NP1 and NP3 from phage genomic DNA and expressed them as further described below. The lysin from phage NP1 was termed PlyPa101, and the lysin from phage NP3 was termed PlyPa102. Through additional searches of *P. aeruginosa* genomes and screening we identified PlyPa103- a homologue of PlyPa101. Purified proteins were tested for their ability to kill *P. aeruginosa* and *Klebsiella* spp. Ply Pal 0 1 and PlyPa103 displayed killing for both species, while PlyPa102 did not displayed detectable killing activity. But like all lysins described herein, non-killing lysins could be used in diagnostic approaches. Killing results for PlyPa101 and PlyPa103 are described further below in Example 2.

As used herein and in the appended claims, the singular forms "a", "and" and "the" include plural references unless the context clearly dictates otherwise.

With respect to this disclosure, if appearing herein, the following terms shall have the definitions as provided and set out below and in this section. Any other terms, including all technical and scientific terms used herein have the same meaning as commonly understood by one of ordinary skill in the art to which this disclosure pertains.

The terms "Pseudomonas lysin(s)", as used throughout the present application and claims refer to proteinaceous material including single or multiple proteins, and extends to those proteins having the amino acid sequence data described herein, including but not necessarily limited to those presented in the Tables, and the profile of activities set forth herein and in the Claims. Accordingly, proteins displaying substantially equivalent or altered activity are likewise contemplated. These modifications may be deliberate, for example, such as modifications obtained through site-directed mutagenesis, or may be accidental, such as those obtained through mutations in hosts that are producers of the complex or its named subunits. In certain embodiments, any amino acid sequence herein can be modified from its naturally occurring sequence to include, for example, additional or fewer amino acids. In embodiments, a lysin described herein is provided in a fusion protein. In embodiments, a polypeptide described herein includes amino acid sequences that originate via being encoded by or as a result of recombinant expression vectors. As non-limiting examples, any lysin amino acid sequence of recombinant protein lysin described herein may contain the sequence of GPVD (SEQ ID NO:27), wherein for example, GP (Glycine Proline) is from a 3C cleavage site and VD (Valine Aspartic acid) is encoded by a SalI restriction site, but other residual amino acids from expression vectors and production of the lysins are included. In one embodiment, a fusion protein comprising a lysin also comprises an anti-microbial peptide (AMP). Many suitable AMPs are known in the art and can be adapted for use with the lysins of this disclosure. See, for example, Lewies A. et al., Probiotics Antimicrob Proteins. 2018 Sep 18. doi: 10.1007/s12602-018-9465-0. As some non-limiting examples, in addition to the AMPs shown in Table 1, suitable AMPs include: Arenicin-1, Cryptdin 2, Nisin Z, Nisin, CAMA, Brevinin-2 CE, Human beta defensin 3, LL-37, Nisin, LL-37, and Nisin Z. Source organisms and sequences of each of these AMPs are known in the art.

### Polypeptides and Lytic Enzymes

A "lytic enzyme" includes any bacterial cell wall lytic enzyme that kills one or more bacteria under suitable conditions and during a relevant time period. Examples of lytic enzymes include, without limitation, various amidase cell wall lytic enzymes.

A "Pseudomonas enzyme" includes a lytic enzyme that is capable of killing at least one or more Pseudomonas bacteria under suitable conditions and during a relevant time period.

A "bacteriophage lytic enzyme" refers to a lytic enzyme encoded by a bacteriophage gene or a synthesized lytic enzyme with a similar protein structure that maintains a lytic enzyme functionality.

A lytic enzyme is capable of specifically cleaving bonds that are present in the peptidoglycan of bacterial cells to disrupt the bacterial cell wall. It is also currently postulated that the bacterial cell wall peptidoglycan is highly conserved among most bacteria, and cleavage of only a few bonds may disrupt the bacterial cell wall. The bacteriophage lytic enzyme may be an amidase, although other types of enzymes are possible. In embodiments, the lysins described herein are lysozymes, which may be referred to lysins PlyPa01-PlyPa96, or they are transglycosylases, which may be referred to lysins PlyPa101-PlyPa103.

A "lytic enzyme genetically coded for by a bacteriophage" includes a polypeptide capable of killing host bacteria, for instance by having at least some cell wall lytic activity against the host bacteria. The polypeptide may have a sequence that encompasses native sequence lytic enzyme and variants thereof. The polypeptide may be isolated from a variety of sources, such as from a bacteriophage ("phage"), or prepared by recombinant or synthetic methods, for which many suitable techniques are known in the art. The polypeptide may comprise a binding portion or a charged portion at the carboxyl terminal side and may be characterized by an enzyme activity capable of cleaving cell wall peptidoglycan (such as an amidase or transglycosylase activity to act on these bonds in the peptidoglycan) at the amino terminal side. Lytic enzymes have been described which include multiple enzyme activities, for example two enzymatic domains, such as PlyGBS lysin. Generally speaking, a lytic enzyme may be between 25,000 and 35,000 daltons in molecular weight and comprise a single polypeptide chain; however, this can vary depending on the enzyme chain. The molecular weight can be determined by assay on denaturing sodium dodecyl sulfate gel electrophoresis and comparison with molecular weight markers.

A "native sequence phage associated lytic enzyme" includes a polypeptide having the same amino acid sequence as an enzyme derived from a bacteriophage. Such native sequence enzyme can be isolated from a phage lysate or can be produced by recombinant or synthetic means.

The term "native sequence enzyme" encompasses naturally occurring forms (for example, alternatively spliced or altered forms) and naturally-occurring variants of the enzyme. In one embodiment of the disclosure, the native sequence enzyme is a mature or full-length polypeptide that is genetically coded for by a gene from a bacteriophage specific for *Pseudomonas aeruginosa.*

"A variant sequence lytic enzyme" includes a lytic enzyme characterized by a polypeptide sequence that is different from that of a lytic enzyme, but retains functional activity. The lytic enzyme can, in some embodiments, be genetically coded for by a bacteriophage specific for *Pseudomonas aeruginosa* and other bacteria as described herein having a particular amino acid sequence identity with the lytic enzyme sequence(s) hereof, as in Table 1.

"Percent amino acid sequence identity" with respect to the phage associated lytic enzyme sequences identified is defined herein as the percentage of amino acid residues in a candidate sequence that are identical with the amino acid residues in the phage associated lytic enzyme sequence, after aligning the sequences in the same reading frame and introducing gaps, if necessary, to achieve the maximum percent sequence identity, and not considering any conservative substitutions as part of the sequence identity.

"Percent nucleic acid sequence identity" with respect to the phage associated lytic enzyme sequences identified herein is defined as the percentage of nucleotides in a candidate sequence that are identical with the nucleotides in the phage associated lytic enzyme sequence, after aligning the sequences and introducing gaps, if necessary, to achieve the maximum percent sequence identity.

To determine the percent identity of two nucleotide or amino acid sequences, the sequences are aligned for optimal comparison purposes (e.g., gaps may be introduced in the sequence of a first nucleotide sequence). The nucleotides or amino acids at corresponding nucleotide or amino acid positions are then compared. When a position in the first sequence is occupied by the same nucleotide or amino acid as the corresponding position in the second sequence, then the molecules are identical at that position. The percent identity between the two sequences is a function of the number of identical positions shared by the sequences (i.e., % identity=# of identical positions/total # of positions × 100).

The term "altered lytic enzymes" includes shuffled and/or chimeric lytic enzymes.

In as much the lysin polypeptide sequences and nucleic acids encoding the lysin polypeptides are provided herein, the lytic enzyme(s)/polypeptide(s) may be produced via the isolated gene for the lytic enzyme from the phage genome, putting the gene into a transfer vector, and cloning said transfer vector into an expression system, using standard methods of the art, including as exemplified herein. The lytic enzyme(s) or polypeptide(s) may be truncated, chimeric, shuffled or "natural," and may be in combination. An "altered" lytic enzyme can be produced in a number of ways. In one embodiment, a gene for the altered lytic enzyme from the phage genome is put into a transfer or movable vector, such as a plasmid, and the plasmid is cloned into an expression vector or expression system. The expression vector for producing a lysin polypeptide or enzyme of the disclosure may be suitable for *E*. *coli, Bacillus, Streptomyces,* and others that will be apparent to those skilled in the art. The vector system may also be a cell free expression system. All of these methods of expressing a gene or set of genes are known in the art.

A "chimeric protein" or "fusion protein" comprises all or a biologically active part of a polypeptide of this disclosure operably linked to a heterologous polypeptide. Chimeric proteins or peptides are produced, for example, by combining two or more proteins having two or more active sites. Chimeric protein and peptides can act independently on the same or different molecules, and hence have a potential to treat two or more different bacterial infections at the same time. Chimeric proteins and peptides also may be used to treat a bacterial infection by cleaving the cell wall in more than one location, thus potentially providing more rapid or effective (or synergistic) killing from a single lysin molecule or chimeric peptide.

A "heterologous" region of a DNA construct or peptide construct is an identifiable segment of DNA within a larger DNA molecule or peptide within a larger peptide molecule that is not found in association with the larger molecule in nature. One non-limiting example of a heterologous coding sequence is a construct where the coding sequence itself is not found in nature (e.g., a cDNA where the genomic coding sequence contains introns, or synthetic sequences having codons different than the native gene).

The term "operably linked" means that the polypeptide of the disclosure and the heterologous polypeptide are fused in-frame. The heterologous polypeptide can be fused to the N-terminus or C-terminus of the polypeptide of the disclosure. Chimeric proteins are produced enzymatically by chemical synthesis, or by recombinant DNA technology. In embodiments, a fusion protein of this disclosure comprises a GST fusion protein in which the polypeptide of the disclosure is fused to the C-terminus of a GST sequence. Such a chimeric protein can facilitate the purification of a recombinant polypeptide of the disclosure.

In another embodiment, the chimeric protein or peptide contains a heterologous signal sequence at its N-terminus. For example, the native signal sequence of a polypeptide of the disclosure can be removed and replaced with a signal sequence from another protein.

The fusion protein can combine a lysin polypeptide with a protein or polypeptide of having a different capability, or providing an additional capability or added character to the lysin polypeptide. The fusion gene can be synthesized by conventional techniques, many suitable techniques for which are known in the art.

As used herein, shuffled proteins or peptides, gene products, or peptides for more than one related phage protein or protein peptide fragments may been randomly cleaved and reassembled into a more active or specific protein. Shuffling can be used to create a protein that is more active, for instance up to 10 to 100 fold more active than the template protein. The template protein is selected among different varieties of lysin proteins. The shuffled protein or peptides constitute, for example, one or more binding domains and one or more catalytic domains. Each binding or catalytic domain is derived from the same or a different phage or phage protein.

A signal sequence of a polypeptide can facilitate transmembrane movement of the protein and peptides and peptide fragments by facilitating secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the disclosure can pertain to the described polypeptides having a signal sequence, as well as to the signal sequence itself and to the polypeptide in the absence of the signal sequence (i.e., the cleavage products). A nucleic acid sequence encoding a signal sequence of the disclosure can be operably linked in an expression vector to a protein of interest, such as a protein which is ordinarily not secreted or is otherwise difficult to isolate. The signal sequence directs secretion of the protein, and the signal sequence is subsequently or concurrently cleaved. The protein can then be readily purified from the extracellular medium by art-recognized methods. Alternatively, the signal sequence can be linked to a protein of interest using a sequence which facilitates purification.

Unless specified to the contrary, it is intended that every maximum numerical limitation given throughout this description includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein

The smallest polypeptide (and associated nucleic acid that encodes the polypeptide) that can be expected to function in embodiments of this disclosure, and is useful for some embodiments may be 20, 25, 30, 35, 40, 45, 50, 55, 60, 65, 75, 85, or 100 amino acids long, or longer.

Biologically active portions of a protein or peptide fragment of the embodiments, as described herein, include polypeptides comprising amino acid sequences sufficiently identical to or derived from the amino acid sequence of the phage protein of the disclosure, which include fewer amino acids than the full length protein of the phage protein and exhibit at least one activity of the corresponding full-length protein. Typically, biologically active portions comprise a domain or motif with at least one activity of the corresponding protein. A biologically active portion of a protein or protein fragment of the disclosure can be a polypeptide which is, for example, 25, 50, 100 more amino acids in length. Moreover, other biologically active portions, in which other regions of the protein are deleted, or added can be prepared by recombinant techniques and evaluated for one or more of the functional activities of the native form of a polypeptide of the embodiments.

In certain non-claimed embodiments, the disclosure includes homologous proteins. Homology in certain non-claimed embodiments is at least 50%, 65%, 75%, 80%, 85%. In certain embodiment, homology is at least 90%, 95%, 97%, 98%, or at least 99% compared to the lysin polypeptides provided herein, including as set out in Table 1. These percent homology values do not include alterations due to conservative amino acid substitutions.

The amino acid residues described herein are preferred to be in the "L" isomeric form. However, residues in the "D" isomeric form can be substituted for any L-amino acid residue, as long as the desired functionality is retained by the polypeptide. NH₂ refers to the free amino group present at the amino terminus of a polypeptide. COOH refers to the free carboxy group present at the carboxy terminus of a polypeptide.

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence may indicate a peptide bond to a further sequence of one or more amino-acid residues.

The term "specific" may be used to refer to the situation in which one member of a specific binding pair will not show significant binding to molecules other than its specific binding partner(s).

The term "comprise" generally used in the sense of include, that is to say permitting the presence of one or more features or components.

The term "consisting essentially of" refers to a product, particularly a peptide sequence, of a defined number of residues which is not covalently attached to a larger product. In the case of the peptide of the disclosure hereof, those of skill in the art will appreciate that minor modifications to the N- or C-terminal of the peptide may however be contemplated, such as the chemical modification of the terminal to add a protecting group or the like, e.g. the amidation of the C-terminus.

The term "isolated" refers to the state in which the lysin polypeptide(s) of the disclosure, or nucleic acid encoding such polypeptides will be, in accordance with the present disclosure. Polypeptides and nucleic acid will be free or substantially free of material with which they are naturally associated such as other polypeptides or nucleic acids with which they are found in their natural environment, or the environment in which they are prepared (e.g. cell culture) when such preparation is by recombinant DNA technology practiced in vitro or in vivo. Polypeptides and nucleic acid may be formulated with diluents or adjuvants and still for practical purposes be isolated--for example the polypeptides will normally be mixed with polymers or mucoadhesives or other carriers, or will be mixed with pharmaceutically acceptable carriers or diluents, when used in diagnosis or therapy.

### Nucleic Acids

Nucleic acids capable of encoding the lysin polypeptide(s) of the disclosure are provided herein and constitute an aspect of the disclosure. Representative nucleic acid sequences in this context are polynucleotide sequences coding for the polypeptide Table 1, and sequences that hybridize, under stringent conditions, with complementary sequences of the DNA of the Table 1 sequence(s). The disclosure includes all polynucleotide sequences, including DNA and RNA, that encode the polypeptides described herein.

A "signal sequence" can be included before the coding sequence. This sequence encodes a signal peptide, N-terminal to the polypeptide, that communicates to the host cell to direct the polypeptide to the cell surface or secrete the polypeptide into the media, and this signal peptide is clipped off by the host cell before the protein leaves the cell. Signal sequences can be found associated with a variety of proteins native to prokaryotes and eukaryotes.

As used herein, the terms "restriction endonucleases" and "restriction enzymes" refer to bacterial enzymes, each of which cut double-stranded DNA at or near a specific nucleotide sequence.

### Compositions

Therapeutic or pharmaceutical compositions comprising the lytic enzyme/polypeptide PlyPa91 of the disclosure are described in accordance with the disclosure, as well as related methods of use and methods of manufacture. Therapeutic or pharmaceutical compositions may comprise the lytic polypeptide PlyPa91, and optionally include natural, truncated, chimeric or shuffled lytic enzymes, optionally combined with other components such as a carrier, vehicle, polypeptide, polynucleotide, one or more antibiotics or suitable excipients, carriers or vehicles or another lysin protein with a different cleavage specificity. The disclosure provides therapeutic compositions or pharmaceutical compositions of the lysin of the disclosure, PlyPa91, for use in the killing, alleviation, decolonization, prophylaxis or treatment of gram-positive bacteria, including bacterial infections or related conditions. The disclosure provides therapeutic compositions or pharmaceutical compositions of the lysin of the disclosure, PlyPa91, for use in treating, reducing or controlling contamination and/or infections by gram negative bacteria, particularly including *Pseudomonas aeruginosa,* including in contamination or infection. Compositions are thereby contemplated and provided for therapeutic applications and local or systemic administration. Compositions comprising PlyPa91, are provided herein for use in the killing, alleviation, decolonization, prophylaxis or treatment of gram-negative bacteria, including bacterial infections or related conditions, particularly *Pseudomonas aeruginosa.*

The pharmaceutical composition comprises at least one isolated and/or recombinant lysin polypeptide that is effective to kill bacteria, wherein the lysin polypeptide comprises an amino acid sequence that is 100% identical to an amino acid sequence of a lysin polypeptide of PlyPa91 (SEQ ID NO: 13).

The pharmaceutical composition can contain a complementary agent, including one or more antimicrobial agent and/or one or more conventional antibiotics. In order to accelerate treatment of the infection, the therapeutic agent may further include at least one complementary agent which can also potentiate the bactericidal activity of the lytic enzyme. Antimicrobials act largely by interfering with the structure or function of a bacterial cell by inhibition of cell wall synthesis, inhibition of cell-membrane function and/or inhibition of metabolic functions, including protein and DNA synthesis. Antibiotics can be subgrouped broadly into those affecting cell wall peptidoglycan biosynthesis and those affecting DNA or protein synthesis in gram positive bacteria. Cell wall synthesis inhibitors, including penicillin and antibiotics like it, disrupt the rigid outer cell wall so that the relatively unsupported cell swells and eventually ruptures. Antibiotics affecting cell wall peptidoglycan biosynthesis include: Glycopeptides, which inhibit peptidoglycan synthesis by preventing the incorporation of N-acetylmuramic acid (NAM) and N-acetylglucosamine (NAG) peptide subunits into the peptidoglycan matrix. Available glycopeptides include vancomycin and teicoplanin. Penicillins, which act by inhibiting the formation of peptidoglycan cross-links. The functional group of penicillins, the β.-lactam moiety, binds and inhibits DD-transpeptidase that links the peptidoglycan molecules in bacteria. Hydrolytic enzymes continue to break down the cell wall, causing cytolysis or death due to osmotic pressure. Common penicillins include oxacillin, ampicillin and cloxacillin; and Polypeptides, which interfere with the dephosphorylation of the Css-isoprenyl pyrophosphate, a molecule that carries peptidoglycan building-blocks outside of the plasma membrane. A cell wall-impacting polypeptide is bacitracin.

The complementary agent may be an antibiotic, such as erythromycin, clarithromycin, azithromycin, roxithromycin, other members of the macrolide family, penicillins, cephalosporins, and any combinations thereof in amounts which are effective to synergistically enhance the therapeutic effect of the lytic enzyme. Virtually any other antibiotic may be used with the altered and/or unaltered lytic enzyme. Similarly, other lytic enzymes may be included in the carrier to treat other bacterial infections. Antibiotic supplements may be used in virtually all uses of the enzyme when treating different diseases. The pharmaceutical composition contains a peptide or a peptide fragment of at least one lytic proteins, wherein the lysin polypeptide comprises an amino acid sequence that is 100% identical to an amino acid sequence of a lysin polypeptide of PlyPa91 (SEQ ID NO: 13), with an optional addition of a complementary agents, and a suitable carrier or diluent.

Also provided are compositions containing nucleic acid molecules that, either alone or in combination with other nucleic acid molecules, are capable of expressing an effective amount of a lytic polypeptide PlyPa91 in vivo. Cell cultures containing these nucleic acid molecules, polynucleotides, and vectors carrying and expressing these molecules in vitro or in vivo, are also provided.

Therapeutic or pharmaceutical compositions may comprise lytic polypeptide PlyPa91 combined with a variety of carriers to treat the illnesses caused by the susceptible Gram-negative bacteria. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; glycine; amino acids such as glutamic acid, aspartic acid, histidine, or arginine; monosaccharides, disaccharides, and other carbohydrates including cellulose or its derivatives, glucose, mannose, trehalose, or dextrins; chelating agents such as EDTA; sugar alcohols such as mannitol or sorbitol; counter-ions such as sodium; non-ionic surfactants such as polysorbates, poloxamers, or polyethylene glycol (PEG); and/or neutral salts, e.g., NaCl, KCl, MgCl₂, CaCl₂, and others. Glycerin or glycerol (1,2,3-propanetriol) is commercially available for pharmaceutical use. It may be diluted in sterile water for injection, or sodium chloride injection, or other pharmaceutically acceptable aqueous injection fluid, and used in concentrations of 0.1 to 100% (v/v), preferably 1.0 to 50% more preferably about 20%. DMSO is an aprotic solvent with a remarkable ability to enhance penetration of many locally applied drugs. DMSO may be diluted in sterile water for injection, or sodium chloride injection, or other pharmaceutically acceptable aqueous injection fluid, and used in concentrations of 0.1 to 100% (v/v). The carrier vehicle may also include Ringer's solution, a buffered solution, and dextrose solution, particularly when an intravenous solution is prepared.

The lytic polypeptide PlyPa91 may be included in a liquid form or in a lyophilized state, whereupon it will be solubilized when it meets body fluids such as mucous. The polypeptide/enzyme PlyPa91 may also be in a micelle or liposome.

The effective dosage rates or amounts of an altered or unaltered lytic enzyme/polypeptide PlyPa91 to treat the infection will depend in part on whether the lytic enzyme/polypeptide PlyPa91 will be used therapeutically or prophylactically, the duration of exposure of the recipient to the infectious bacteria, the size and weight of the individual, etc. The duration for use of the composition containing the enzyme/polypeptide PlyPa91 also depends on whether the use is for prophylactic purposes, wherein the use may be hourly, daily or weekly, for a short time period, or whether the use will be for therapeutic purposes wherein a more intensive regimen of the use of the composition may be needed, such that usage may last for hours, days or weeks, and/or on a daily basis, or at timed intervals during the day. Any dosage form employed should provide for a minimum number of units for a minimum amount of time. The concentration of the active micrograms (ug) of enzyme believed to provide for an effective amount or dosage of enzyme may be in the range of about 10 ug/ml to about 10,000 ug/ml of fluid in the wet or damp environment of the nasal and oral passages, and possibly in the range of about 100 ug/ml to about 500 ug/ml. More specifically, time exposure to the active enzyme/polypeptide(s) ug may influence the desired concentration of active enzyme ug per ml. Carriers that are classified as "long" or "slow" release carriers (such as, for example, certain nasal sprays or lozenges) could possess or provide a lower concentration of active (enzyme) ug per ml, but over a longer period of time, whereas a "short" or "fast" release carrier (such as, for example, a gargle) could possess or provide a high concentration of active (enzyme) ug per ml, but over a shorter period of time. The amount of active ug per ml and the duration of time of exposure depend on the nature of infection, whether treatment is to be prophylactic or therapeutic, and other variables. There are situations where it may be necessary to have a much higher unit/ml dosage or a lower ug/ml dosage.

The lytic enzyme/polypeptide PlyPa91 should be in an environment having a pH which allows for activity of the lytic enzyme/polypeptide(s). For example if a human individual has been exposed to another human with a bacterial upper respiratory disorder, the lytic enzyme/polypeptide(s) will reside in the mucosal lining and prevent any colonization of the infecting bacteria. Prior to, or at the time the altered lytic enzyme is put in the carrier system or oral delivery mode, it is preferred that the enzyme be in a stabilizing buffer environment for maintaining a pH range between about 4.0 and about 9.0, more preferably between about 5.5 and about 7.5.

A stabilizing buffer may allow for the optimum activity of the lysin enzyme/polypeptide PlyPa91 . The buffer may contain a reducing reagent, such as dithiothreitol. The stabilizing buffer may also be or include a metal chelating reagent, such as ethylenediaminetetracetic acid disodium salt, or it may also contain a phosphate or citrate-phosphate buffer, or any other buffer. The DNA coding of these phages and other phages may be altered to allow a recombinant enzyme to attack one cell wall at more than two locations, to allow the recombinant enzyme to cleave the cell wall of more than one species of bacteria, to allow the recombinant enzyme to attack other bacteria, or any combinations thereof. The type and number of alterations to a recombinant bacteriophage produced enzyme are incalculable.

A mild surfactant can be included in a therapeutic or pharmaceutical composition in an amount effective to potentiate the therapeutic effect of the lytic enzyme/polypeptide(s) may be used in a composition. Suitable mild surfactants include, inter alia, esters of polyoxyethylene sorbitan and fatty acids (Tween series), octylphenoxy polyethoxy ethanol (Triton-X series), n-Octyl-β-D-glucopyranoside, n-Octyl-β.-D-thioglucopyranoside, n-Decyl-β-D-glucopyranoside, n-Dodecyl-β-D-glucopyranoside, and biologically occurring surfactants, e.g., fatty acids, glycerides, monoglycerides, deoxycholate and esters of deoxycholate or Survanta or other lung surfactant preparations.

Preservatives may also be used and generally comprise about 0.05% to 0.5% by weight of the total composition. The use of preservatives assures that if the product is microbially contaminated, the formulation will prevent or diminish microorganism growth. Some preservatives useful in this disclosure include methylparaben, propylparaben, butylparaben, chloroxylenol, sodium benzoate, DMDM Hydantoin, 3-Iodo-2-Propylbutyl carbamate, potassium sorbate, chlorhexidine digluconate, or a combination thereof.

Pharmaceuticals for use in all embodiments of the disclosure may include antimicrobial agents, anti-inflammatory agents, antiviral agents, local anesthetic agents, corticosteroids, destructive therapy agents, antifungals, and antiandrogens. In the treatment of acne, active pharmaceuticals that may be used include antimicrobial agents, especially those having anti-inflammatory properties such as dapsone, erythromycin, minocycline, tetracycline, clindamycin, and other antimicrobials. In embodiments, the weight percentages for the antimicrobials are 0.5% to 10%.

Local anesthetics include tetracaine, tetracaine hydrochloride, lidocaine, lidocaine hydrochloride, dyclonine, dyclonine hydrochloride, dimethisoquin hydrochloride, dibucaine, dibucaine hydrochloride, butambenpicrate, and pramoxine hydrochloride. In an embodiment, a concentration for local anesthetics is about 0.025% to 5% by weight of the total composition. Anesthetics such as benzocaine may also be used at a concentration of about 2% to 25% by weight.

Corticosteroids that may be used include betamethasone dipropionate, fluocinolone actinide, betamethasone valerate, triamcinolone actinide, clobetasol propionate, desoximetasone, diflorasone diacetate, amcinonide, flurandrenolide, hydrocortisone valerate, hydrocortisone butyrate, and desonide are recommended at concentrations of about 0.01% to 1.0% by weight. Concentrations for corticosteroids such as hydrocortisone or methylprednisolone acetate are from about 0.2% to about 5.0% by weight.

Additionally, the therapeutic composition may further comprise other enzymes, such as the enzyme lysostaphin for the treatment of any *Staphylococcus aureus* bacteria present along with the susceptible gram-negative bacteria. Mucolytic peptides, such as lysostaphin, have been suggested to be efficacious in the treatment of *S. aureus* infections of humans. The use of the lysin and lysostaphin, possibly in combination with antibiotics, can serve as a rapid and effective treatment of mixed bacterial infections. A therapeutic composition may also include mutanolysin, and lysozyme.

Methods of application of the therapeutic composition comprising a lytic enzyme/polypeptide PlyPa91 include, but are not limited to direct, indirect, carrier and special means or any combination of means. Direct application of the lytic enzyme/polypeptide(s) may be by any suitable means to directly bring the polypeptide in contact with the site of infection or bacterial colonization, such as to the nasal area (for example nasal sprays), dermal or skin applications (for example topical ointments or formulations), suppositories, tampon applications, etc. Nasal applications include for instance nasal sprays, nasal drops, nasal ointments, nasal washes, nasal injections, nasal packings, bronchial sprays and inhalers, or indirectly through use of throat lozenges, mouthwashes or gargles, or through the use of ointments applied to the nasal nares, or the face or any combination of these and similar methods of application. The forms in which the lytic enzyme may be administered include but are not limited to lozenges, troches, candies, injectants, chewing gums, tablets, powders, sprays, liquids, ointments, and aerosols.

When the natural and/or altered lytic enzyme/polypeptide PlyPa91 is introduced directly by use of sprays, drops, ointments, washes, injections, packing and inhalers, the enzyme is a liquid or gel environment, with the liquid acting as the carrier. A dry anhydrous version of the altered enzyme may be administered by the inhaler and bronchial spray, although a liquid form of delivery included.

Compositions for treating topical infections or contaminations comprise an effective amount of at least one lytic enzyme, PlyPa91, according to the disclosure and a carrier for delivering at least one lytic enzyme to the infected or contaminated skin, coat, or external surface of a companion animal or livestock. The mode of application for the lytic enzyme includes a number of different types and combinations of carriers which include, but are not limited to an aqueous liquid, an alcohol base liquid, a water soluble gel, a lotion, an ointment, a nonaqueous liquid base, a mineral oil base, a blend of mineral oil and petrolatum, lanolin, liposomes, protein carriers such as serum albumin or gelatin, powdered cellulose carmel, and combinations thereof. A mode of delivery of the carrier containing the therapeutic agent includes, but is not limited to a smear, spray, a time-release patch, a liquid absorbed wipe, and combinations thereof. The lytic enzyme may be applied to a bandage either directly or in one of the other carriers. The bandages may be sold damp or dry, wherein the enzyme is in a lyophilized form on the bandage. This method of application is most effective for the treatment of infected skin. The carriers of topical compositions may comprise semi-solid and gel-like vehicles that include a polymer thickener, water, preservatives, active surfactants or emulsifiers, antioxidants, sun screens, and a solvent or mixed solvent system. U.S. Pat. No. 5,863,560 (Osborne) discusses a number of different carrier combinations which can aid in the exposure of the skin to a medicament. Polymer thickeners that may be used include those known to one skilled in the art, such as hydrophilic and hydroalcoholic gelling agents frequently used in the cosmetic and pharmaceutical industries. The composition sold under the trade name CARBOPOL is one of numerous cross-linked acrylic acid polymers that are given the general adopted name carbomer. These polymers dissolve in water and form a clear or slightly hazy gel upon neutralization with a caustic material such as sodium hydroxide, potassium hydroxide, triethanolamine, or other amine bases. The composition sold under the trade name KLUCEL is a cellulose polymer that is dispersed in water and forms a uniform gel upon complete hydration. Other gelling polymers include hydroxyethylcellulose, cellulose gum, MVE/MA decadiene crosspolymer, PVM/MA copolymer, or a combination thereof.

Compositions comprising the lytic enzyme PlyPa91, or their peptide fragments can be directed to the mucosal lining, where, in residence, they kill colonizing disease bacteria. The mucosal lining, as disclosed and described herein, includes, for example, the upper and lower respiratory tract, eye, buccal cavity, nose, rectum, vagina, periodontal pocket, intestines and colon. Due to natural eliminating or cleansing mechanisms of mucosal tissues, conventional dosage forms are not retained at the application site for any significant length of time.

It may be advantageous to have materials which exhibit adhesion to mucosal tissues, to be administered with one or more phage enzymes and other complementary agents over a period of time. Materials having controlled release capability are particularly desirable, and the use of sustained release mucoadhesives has received a significant degree of attention. J. R. Robinson (U.S. Pat. No. 4,615,697) provides a review of the various controlled release polymeric compositions used in mucosal drug delivery, including a controlled release treatment composition which includes a bioadhesive and an effective amount of a treating agent. The bioadhesive is a water swellable, but water insoluble fibrous, crosslinked, carboxy functional polymer containing (a) a plurality of repeating units of which at least about 80 percent contain at least one carboxyl functionality, and (b) about 0.05 to about 1.5 percent crosslinking agent substantially free from polyalkenyl polyether. While the polymers of Robinson are water swellable but insoluble, they are crosslinked, not thermoplastic, and are not as easy to formulate with active agents, and into the various dosage forms, as the copolymer systems of the present application. Micelles and multilamillar micelles may also be used to control the release of enzyme.

Other approaches involving mucoadhesives which are the combination of hydrophilic and hydrophobic materials, are known. The composition sold under the trade name Orahesive from E.R. Squibb & Co is an adhesive which is a combination of pectin, gelatin, and sodium carboxymethyl cellulose in a tacky hydrocarbon polymer, for adhering to the oral mucosa. However, such physical mixtures of hydrophilic and hydrophobic components eventually fall apart. In contrast, the hydrophilic and hydrophobic domains in this application produce an insoluble copolymer. U.S. Pat. No. 4,948,580, describes a bioadhesive oral drug delivery system. The composition includes a freeze-dried polymer mixture formed of the copolymer poly(methyl vinyl ether/maleic anhydride) and gelatin, dispersed in an ointment base, such as mineral oil containing dispersed polyethylene. U.S. Pat. No. 5,413,792 discloses paste-like preparations comprising (A) a paste-like base comprising a polyorganosiloxane and a water soluble polymeric material which may be present in a ratio by weight from 3:6 to 6:3, and (B) an active ingredient. U.S. Pat. No. 5,554,380 claims a solid or semisolid bioadherent orally ingestible drug delivery system containing a water-in-oil system having at least two phases. One phase comprises from about 25% to about 75% by volume of an internal hydrophilic phase and the other phase comprises from about 23% to about 75% by volume of an external hydrophobic phase, wherein the external hydrophobic phase is comprised of three components: (a) an emulsifier, (b) a glyceride ester, and (c) a wax material. U.S. Pat. No. 5,942,243 describes some representative release materials useful for administering antibacterial agents.

Therapeutic or pharmaceutical compositions can also contain polymeric mucoadhesives including a graft copolymer comprising a hydrophilic main chain and hydrophobic graft chains for controlled release of biologically active agents. The graft copolymer is a reaction product of (1) a polystyrene macromonomer having an ethylenically unsaturated functional group, and (2) at least one hydrophilic acidic monomer having an ethylenically unsaturated functional group. The graft chains consist essentially of polystyrene, and the main polymer chain of hydrophilic monomeric moieties, some of which have acidic functionality. The weight percent of the polystyrene macromonomer in the graft copolymer is between about 1 and about 20% and the weight percent of the total hydrophilic monomer in the graft copolymer is between 80 and 99%, and wherein at least 10% of said total hydrophilic monomer is acidic, said graft copolymer when fully hydrated having an equilibrium water content of at least 90%. Compositions containing the copolymers gradually hydrate by sorption of tissue fluids at the application site to yield a very soft jelly like mass exhibiting adhesion to the mucosal surface. During the period of time the composition is adhering to the mucosal surface, it provides sustained release of the pharmacologically active agent, which is absorbed by the mucosal tissue.

The compositions of this application may optionally contain other polymeric materials, such as poly(acrylic acid), poly,-(vinyl pyrrolidone), and sodium carboxymethyl cellulose plasticizers, and other pharmaceutically acceptable excipients in amounts that do not cause deleterious effect upon mucoadhesivity of the composition.

The dosage forms of the compositions of this disclosure can be prepared by conventional methods. In cases where intramuscular injection is the chosen mode of administration, an isotonic formulation is used. Generally, additives for isotonicity can include sodium chloride, dextrose, mannitol, sorbitol and lactose. In some cases, isotonic solutions such as phosphate buffered saline are preferred. Stabilizers include gelatin and albumin. A vasoconstriction agent can be added to the formulation. The pharmaceutical preparations according to this application are provided sterile and pyrogen free.

A lytic enzyme/polypeptide PlyPa91 of the disclosure may also be administered by any pharmaceutically applicable or acceptable means including topically, orally or parenterally. For example, the lytic enzyme/polypeptide(s) can be administered intramuscularly, intrathecally, subdermally, subcutaneously, or intravenously to treat infections by gram-negative bacteria. In cases where parenteral injection is the chosen mode of administration, an isotonic formulation is used. Generally, additives for isotonicity can include sodium chloride, dextrose, mannitol, sorbitol and lactose. In some cases, isotonic solutions such as phosphate buffered saline are preferred. Stabilizers include gelatin and albumin. A vasoconstriction agent can be added to the formulation. The pharmaceutical preparations according to this application are provided sterile and pyrogen free.

For any lysin, a therapeutically effective dose can be estimated initially either in cell culture assays or in animal models, usually mice, rabbits, dogs, or pigs. The animal model is also used to achieve a desirable concentration range and route of administration. Such information can then be used to determine useful doses and routes for administration in humans. The exact dosage is chosen by the individual physician in view of the patient to be treated. Dosage and administration are adjusted to provide sufficient levels of the active moiety or to maintain the desired effect. Additional factors which may be taken into account include the severity of the disease state, age, weight and gender of the patient; diet, desired duration of treatment, method of administration, time and frequency of administration, drug combination(s), reaction sensitivities, and tolerance/response to therapy. Long acting pharmaceutical compositions might be administered every 3 to 4 days, every week, or once every two weeks depending on half-life and clearance rate of the particular formulation.

The effective dosage rates or amounts of the lytic enzyme/polypeptide(s) to be administered parenterally, and the duration of treatment will depend in part on the seriousness of the infection, the weight of the patient, particularly human, the duration of exposure of the recipient to the infectious bacteria, and a variety of a number of other variables. The composition may be administered anywhere from once to several times a day, and may be administered for a short or long term period. The usage may last for days or weeks. Any dosage form employed should provide for a minimum number of units for a minimum amount of time. The concentration of the active units of enzymes believed to provide for an effective amount or dosage of enzymes may be selected as appropriate. The amount of active units per ml and the duration of time of exposure depend on the nature of infection, and the amount of contact the carrier allows the lytic enzyme(s)/polypeptide(s) to have.

### Methods and Assays

The bacterial killing capability exhibited by the lysin polypeptide PlyPa91 of the disclosure provides for various methods based on the antibacterial effectiveness of the polypeptide(s) of the disclosure. Thus, the present disclosure contemplates antibacterial methods, including methods for killing of Gram-negative bacteria, for reducing a population of Gram-negative bacteria, for treating or alleviating a bacterial infection, for treating a human subject exposed to a pathogenic bacteria, and for treating a human subject at risk for such exposure.

In embodiments, the disclosure includes treatment, decolonization, and/or decontamination of bacteria, cultures or infections or in instances wherein *Pseudomonas aeruginosa* bacteria is suspected, present, or may be present. This disclosure may also be used to treat gastrointestinal disorders, particularly in a human.

Also described is a method for treating *Pseudomonas aeruginosa* infection, carriage or populations comprises treating the infection with a therapeutic agent comprising an effective amount of the lytic enzyme/polypeptide of the disclosure, PlyPa91. In the methods of the disclosure, the lysin polypeptide of the present disclosure, PlyPa91, is useful and capable in prophylactic and treatment methods directed against gram-negative bacteria, particularly *Pseudomonas aeruginosa* infections or bacterial colonization.

The disclosure includes methods of treating or bacterial infections, related infections or conditions, including antibiotic-resistant bacteria, particularly including wherein the bacteria or a human subject infected by or exposed to the particular bacteria, or suspected of being exposed or at risk, is contacted with or administered an amount of isolated and/or recombinant lysin polypeptide PlyPa91 of the disclosure effective to kill the particular bacteria. Thus, PlyPa91 is contacted or administered so as to be effective to kill the relevant bacteria or otherwise alleviate or treat the bacterial infection.

The term "agent" means any molecule, including polypeptides, antibodies, polynucleotides, chemical compounds and small molecules. In particular the term agent includes compounds such as test compounds, added additional compound(s), or lysin enzyme compounds.

The term "preventing" or "prevention" refers to a reduction in risk of acquiring or developing a disease or disorder (i.e., causing at least one of the clinical symptoms of the disease not to develop) in a subject that may be exposed to a disease-causing agent, or predisposed to the disease in advance of disease onset.

The term "prophylaxis" is related to and encompassed in the term "prevention", and refers to a measure or procedure the purpose of which is to prevent, rather than to treat or cure a disease.

"Therapeutically effective amount" means that amount of a lysin or derivative as described herein that will elicit the biological or medical response of a subject that is being sought by a medical doctor or other clinician. In particular, with regard to gram-negative bacterial infections and growth of gram-negative bacteria, the term "effective amount" is intended to include an effective amount of a compound or agent that will bring about a biologically meaningful decrease in the amount of or extent of infection of gram-negative bacteria, including having a bactericidal effect. The phrase "therapeutically effective amount" is used herein to mean an amount sufficient to prevent, and preferably reduce by at least about 30 percent, more preferably by at least 50 percent, most preferably by at least 90 percent, a clinically significant change in the amount of infectious bacteria, or other feature of pathology such as for example, elevated fever or white cell count as may attend its presence and activity.

The term "treating" or "treatment" of any disease or infection refers, in one embodiment, to ameliorating the disease or infection (i.e., killing the infectious gram negative bacteria or reducing the manifestation, extent or severity of at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or infection, either physically, (e.g., stabilization of a discernible symptom), physiologically, (e.g., stabilization of a physical parameter), or both. In a further embodiment, "treating" or "treatment" relates to slowing the progression of a disease or reducing an infection. Methods of treatment are not claimed.

The phrase "pharmaceutically acceptable" refers to molecular entities and compositions that are physiologically tolerable and do not typically produce an allergic or similar untoward reaction, such as gastric upset, dizziness and the like, when administered to a human.

The term "bactericidal" refers to capable of killing bacterial cells.

The phrase "pharmaceutically acceptable" refers to molecular entities and compositions that are physiologically tolerable and do not typically produce an allergic or similar untoward reaction, such as gastric upset, dizziness and the like, when administered to a human.

The phrase "therapeutically effective amount" is used herein to mean an amount sufficient to prevent, and reduce by at least about 30 percent, by at least 50 percent, or by at least 90 percent, a clinically significant change in the S phase activity of a target cellular mass, or other feature of pathology such as for example, elevated blood pressure, fever or white cell count as may attend its presence and activity.

A pharmaceutical composition for use in a method of killing bacteria on or in an individual, the method comprising contacting the bacteria with an effective amount of the pharmaceutical composition, is disclosed herein.

The diagnostic, prophylactic and therapeutic possibilities and applications that are raised by the recognition of and isolation of the lysin polypeptide(s) of the disclosure, derive from the fact that the polypeptides of the disclosure cause direct and specific effects (e.g. killing) in susceptible bacteria. Thus, the polypeptides of the disclosure may be used to eliminate, characterize, or identify the relevant and susceptible bacteria. Thus, a diagnostic method of the present disclosure may comprise examining a cellular sample or medium for the purpose of determining whether it contains susceptible bacteria, or whether the bacteria in the sample or medium are susceptible by use of an assay including an effective amount of one or more lysin polypeptide(s) and any suitable technique for characterizing one or more cells in the sample, or for determining whether or not cell lysis has occurred or is occurring. Patients capable of benefiting from this method include those suffering from an undetermined infection, a recognized bacterial infection, or suspected of being exposed to or carrying a particular bacteria. A fluid, food, medical device, composition or other such sample which will come in contact with a subject or patient may be examined for susceptible bacteria or may be eliminated of relevant bacteria. In one such aspect a fluid, food, medical device, composition or other such sample may be sterilized or otherwise treated to eliminate or remove any potential relevant bacteria by incubation with or exposure to one or more lytic polypeptide(s) of the disclosure.

In one embodiment, the lytic polypeptide(s) of the disclosure complex(es) with or otherwise binds or associates with relevant or susceptible bacteria in a sample and one member of the complex is labeled with a detectable label. That a complex has formed and, if desired, the amount thereof, can be determined by known methods applicable to the detection of labels. The labels most commonly employed for these studies are radioactive elements, enzymes, chemicals which fluoresce when exposed to ultraviolet light, and others. A number of fluorescent materials are known and can be utilized as labels. These include, for example, fluorescein, rhodamine, auramine, Texas Red, AMCA blue and Lucifer Yellow. The radioactive label can be detected by any of the currently available counting procedures. Enzyme labels are likewise useful, and can be detected by any of the presently utilized colorimetric, spectrophotometric, fluorospectrophotometric, amperometric or gasometric techniques. The enzyme is conjugated to the selected particle by reaction with bridging molecules such as carbodiimides, diisocyanates, glutaraldehyde and the like. Many enzymes which can be used in these procedures are known and can be utilized. In a different embodiment an enzyme could be used to lyse the target bacteria, resulting in the release of a detectable substance including but not limited to ATP. Release of such substance as a result of the application of a lysin could be used to detect the presence of susceptible bacteria and/or to determine whether a bacterium isolated from a patient belongs to the group of organisms for which the lysin is effective.

In Table 1, differences in amino acid sequences for those lysins with the designation PlyPA101 and those with PlyPA101 and a suffix can be readily determined by comparison of the PlyPA101 sequence with those PlyPA101 sequences with a suffix in the Sequence code column. Fusion proteins with a suffice represent an additional amino acid sequence added to the C-terminal end of PlyPA101. The additional amino acid sequences are various anti-microbial peptides (AMPs). A wide variety of other AMPs can be substituted for these representative embodiments, and can also be included on any other lysin described herein. In certain embodiments a flexible linker may be included between the lysin and the fused AMP. In certain embodiments the flexible linker may comprise a glycine and serine rich sequence such as GGGSGGGSGGGG (SEQ ID NO:28). The invention relates to PlyPa91, (SEQ ID NO: 13), and the other disclosed lysins serve as comparative examples.

**Table 1. Representative amino acid sequences of Pseudomonas aeruginosa lysins of this disclosure.**

| Sequence code | Lysin name | Sequence |
|---|---|---|
| PL1 (SEQ ID NO:1) | PlyPA01 | MRTSQRGIDLIKSFEGLRLSAYQDSVGVWTIGYGTTRGVTRYMTITV EQAERMLSNDLQRFEPELDRLVKVPLNQNQWDALMSFVYNLGAAN LESSTLRRLLNAGDYAGAAEQFLRWNKAGGKVLPGLVRRRASEREL FLGAA |
| PL2 (SEQ ID NO:2) | PlyPA02 | MRTSQRGIDLIKSFEGLRLSAYQDSVGVWTIGYGTTRGVTRYMTITV EQAERMLSNDLQRFEPELDRLVKVPLNQNQWDALMSFVYNLGAAN LASSTLLKLLNKGDYQGAADQFPRWVNAGGKRLEGLVKRRAAERA LFLESLS |
| PL1 (SEQ ID NO:3) | PlyPA03 | MRTSQRGIDLIKGFEGLRLSAYQDSVGVWTIGYGTTRGVTRYMTITV EQAERMLSNDLRRFEPELDRLVKAPLNQNQWDALMSFVYNLGAAN LASSTLLKLLNKGDYQGAADQFPRWVNAGGKRLEGLVKRRAAERV LFLEPLS |
| PL1 (SEQ ID NO:29) | PlyPA04 | MRTSQRGIDLIKSFEGLRLSAYQDSVGVWTIGYGTTRGVTRYMTITV EQAERMLANDIQRFEPELDKLVKVPLNQNQWDALMSFVYNLGSAN LASSTLLKLLNKGDYRGAADQFPRWVNAGGKRLEGLVKRRAAERA LFLEPLS |
| PL1 (SEQ ID NO:4) | PlyPA19 | MRTSQRGIDLIKSFEGLRLSAYQDSVGVWTIGYGTTRGVTRYMTITV EQAERMLSNDIQRFEPEMDKLVKVPLNQNQWDALMSFVYNLGAAN LASSTLLKLLNKGDYQGAADQFPRWVNAGGKRLEGLVKRRAAERV LFLEPLS |
| PL21 (SEQ ID NO:5) | PlyPA21 | MRTSQRGIDLIKSFEGLRLSAYQDSVGVWTIGYGTTRGVTRYMTITV EQAERMLSNDLQRFEPELDRLVKVPLNQNQWDALMSFVYNLGAAN LASSTLLKLLNRGDYQGAADQFSRWVNAGGKRLGGLVKRRAAERV LFLEPLS |
| PL29 (SEQ ID NO:6) | PlyPA29 | MRTSQRGIDLIKSFEGLRLSAYQDSVGVWTIGYGTTRGVTRYMTITV EQAERMLSNDLQRFEPELDRLVKVALNQNQWDALMSFVYNLGAAN LASSTLLKLLNKGDYQGAAEQFPRWVNAGRKRLEGLVKRRAAERA LFLEPLS |
| PL40 (SEQ ID NO:7) | PlyPA40 | MNISKAGLDLIKEFEGLRLSAYQDSVGVWTIGYGHTRTAKRGMSITS DQADALLIADLADAEDDVERYVRQDMRQNEFDALVSLVFNIGGSNF SRSTMLRLINEKAEAWKIGAEFLKWVYAKGRKLPGLERRRLAERNL YLKGA |
| PL49 (SEQ ID NO:8) | PlyPA49 | MKLPRKLTAAGGALALAAALVTPFEGRSLVAYRDPVGIPTICEGITA GVRMGDMATPAECDALLKRELQRAVDAVDRQVLVPLPDTRRAALA SFVYNVGEGQLARSTLLRKLNAGDVRGACAELSRWVYAGGKKLGG LVRRRAAERELCEVGL |
| PL58 (SEQ ID NO:9) | PlyPA58 | MKLPPKLAAGGGVLALAAALVAPFEGRSLVAYLDPVGIPTICEGITA GVRMGDRATQAECDALLERELQRAVDAVDRQVLVPLPDTRRAALG SFVYNVGEGQLARSTLLRKLNAGDVRGACAELSRWVYAGGKKLGG LVRRRAAERELCEIGL |
| PL64 (SEQ ID NO:10) | PlyPA64 | MKTWQRVTGALAIASALVAAHEGRSLVAYIDPVGIPTICEGITAGVR LGDRATPQQCDALLETEVRKSLSSVERLATVQMPDTRKAALASFVY NVGETQFSRSTLLRKLNAGDVKGACAELSRWVYAGGKVYKGLVNR RKAERELCERGL |
| PL78 (SEQ ID NO:11) | PlyPA78 | MIRPPQRRTVAALTLSAAALVGIVLHEGYTDRAVIPVKGDVPTIGFG TTTGVKLGDTTTPPKALARALTDVQQFEGALKQCVTVPLAQHEYDA LVSFSYNVGSRAFCQSTLVRKLNAEDYAGACAELLRWRFFQGKDCA QPANARLCGGLVTRREAEYRQCIGETP |
| PL80 (SEQ ID NO:12) | PlyPA80 | MAKRFEGFHRVPKSDPLRRAHPYICPAGYWTIGYGRLCKPDHPPISE DEGEAYLRQDLRTALAATLRYCPVLATEPEGRLAAIADFTFNLGAG RLQTSTLRRRINQRDWPAAATELRRWVYGGGKVLPGLVTRREAEV ALLLRNA |
| PL91 (SEQ ID NO:13) | PlyPA91 | MKGKVIGGSAAAVIALAAAALVKPWEGYSPTPYIDMVGVATHCYG DTSRADKAVYTEQECAEKLNSRLGSYLTGISQCIKVPLREREWAAVL SWTYNVGVGAACRSTLVGRINAGQPAASWCPELDRWVYAGGKRV QGLVNRRAAERRMCEGRS |
| PL92 (SEQ ID NO:14) | PlyPA92 | MLAVLAGTGFTLTSQDLPAPIERAAITAGLMVLTPEMEGTRFKAYPD TGGVWTICTGRTQGVKRGDQATPDECAAYLRADLGASVDFVLRER PKVSLLCKVAIADMHYNTGPGAVGRSTLLVKAKAGDQVGAAEQFR RWVYVGGQDCRLASSNCGGIINRREIQRSLCLVNQ |
| PL96 (SEQ ID NO:15) | PlyPA96 | MASRKYLSAAVLALIAASASAPAIMDQFIREKEGESLKAYQDGARV WTICNGKTAGVTRSTTMTKAECDAWRRTEIGQRLEFVHSIITVRMSE PAWAGVGSWCFNVGNKACAGSTAVRLLNAGNQPAGCRAMLSWRF ITRDGKKVDCSTPQPYCSGVWERRQGEAELCSL |
| NP1 (SEQ ID NO:16) | PlyPA101 | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALEL |
| NP3 (SEQ ID NO:17) | PlyPA102 | MKITKDVLITGTGCTTDRAIKWLDDVQAAMDKFHIESPRAIAAYLA NIGVESGGLVSLVENLNYSAQGLANTWPRRYAVDPRVRPYVPNALA NRLARNPVAIANNVYADRMGNGCEQDGDGWKYRGRGLIQLTGKS NYSLFAEDSGMDVLEKPELLETPAGASMSSAWFFWRNRCIPMAESN NFSMVVKTINGAAPNDANHGQLRINRYLKTIAAINQGS |
| (SEQ ID NO:18) | PlyPa103 | MAWSAKVSQAFCDRVIWIAASLGMPADGADWLMACIAWETGETFS PSVRNGAGSGATGLIQFMPATARGLGTTTDELARMTPEQQLDYVYR YFLPYRGRLKSLADTYMAILWPAGIGRALDWALWDSTSRPTTYRQN AGLDINRDGVITKAEAAAKVQAKLDRGLQPQFRRAAA |
| (SEQ ID NO:19) | PlyPa101-AMP 1 (LL-37) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGLLGDFFRKSKEKIGKEFKRIVQRIKDFLRNLVPRTES |
| (SEQ ID NO:20) | PlyPa101-AMP2 (LALF) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGDHECHYRIKPTFRRLKWKYKGKFWCPS |
| (SEQ ID NO:21) | PlyPa101-AMP3 (RI-18) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGRKKTRKRLKKIGKVLKWI |
| (SEQ ID NO:22) | PlyPa101-AMP4 (WLBU) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGRRWVRRVRRWVRRVVRVVRRWVRR |
| (SEQ ID NO:23) | PlyPa101-AMP5 (RP-1) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGALYKKFKKKLLKSLKRKG |
| (SEQ ID NO:24) | PlyPa101-AMP6 (Pexigana n) | MKLAWGKKVDQAFRDKVFAICDGFKWNRETHASWLMSCMAFESG ETFSPSVRNAAGSGATGLIQFMPRTAQGLGTSTAELAAMSAVDQLD YVQKYFRPYASRIGTLSDMYMAILMPKFVGQPEDSVLFLDPKISYRQ NAGLDANRDGKITKAEAASKVRAKFDKGMLDRFALELGTGGGSGG GSGGGGIGKFLKKAKKFGKAFVKILKK |

Linker sequences are in italics, AMP sequences are in bold.

The disclosure may be better understood by reference to the following non-limiting Examples, which are provided as exemplary of the disclosure. The following examples are presented in order to more fully illustrate the embodiments of the disclosure. The scope of the invention is defined by the appended claims.

### EXAMPLE 1

This Example provides a description of results for lysins initially identified using phylogenetic approach.

### Example 1.1 - Identification of P. aeruginosa phage lysins based on homology search

To identify phage lysins with bacteriolytic activity against *P. aeruginosa* we first performed a BLAST search for genes with homology to the *Acinetobacter baumanii* phage lysin PlyF307 (Lood, Winer et al. 2015) within *P*. *aeruginosa* genomes available in the NCBI database, resulting in over 100 hits. These were aligned using the MUSCLE algorithm and a phylogenic tree was created, revealing 11 major homology groups. We then selected one lysin sequence in each of the 11 major groups and produced a synthetic DNA for each lysin for subsequent protein expression. To screen for catalytically active lysins, these 11 candidates (PlyPa01, PlyPa02, PlyPa40, PlyPa49, PlyPa58, PlyPa64, PlyPa78, PlyPa80, PlyPa91, PlyPa92, PlyPa96) were inserted into pAR533, a pBAD24-based plasmid with an altered multi-cloning site. In one approach, strains containing the expression plasmid were grown on plates containing arabinose to promote expression of the protein. Lysins were released from the streaked cells by exposure to chloroform vapor, and catalytic activity was evaluated by overlaying the plate with soft agar containing autoclaved *P. aeruginosa,* and examining the formation of clearing zones around the streaked cells (Fig. 12). In a different approach, an induced lysate of the different strains was applied to a plate containing soft agar with autoclaved *P. aeruginosa,* and the degree of lysis was evaluated (for a representative image see Fig. 13). A summary of the results obtained in this analysis is presented in Table 2. The results of the two methods were consistent, with one exception (activity for PlyPa58 was only observed using the crude lysate method). Lysins demonstrating peptidoglycan hydrolase activity in both initial analysis (PlyPa01, PlyPa02, PlyPa40, PlyPa49, PlyPa64, PlyPa91, PlyPa96) were characterized further.

### Example 1.2 - Evaluation of lysin killing activity against P. aeruginosa

To evaluate the killing activity of the lysins against live *P. aeruginosa* cells, we produced 3C-cleavable hexahistidine tag fusion protein versions for lysins that demonstrated catalytic activity against autoclaved *Pseudomonas.* These lysins were purified by metal ion affinity chromatography and separated on a gel (Fig. 14) and the hexahistidine tag was cleaved by 3C protease (an example is presented in Fig. 15). In this manner, the final purified and cleaved product contained only 4 additional N-terminal amino acids (Gly, Pro,Val and Asp) compared to the native molecule. We evaluated the ability of purified and 3C-cleaved lysins to kill log-phase *P. aeruginosa* strain PA01 (Fig. 1A). Log-phase PA01 cells were incubated with different lysin dilutions at 37°C for 1 hour. All lysins demonstrated killing activity to some extent, however PlyPa01, PlyPa02, PlyPa91, and PlyPa96 had better activity compared to the other lysins. Of these PlyPa01 and PlyPa02 were more closely related to the *A. baumannii* lysin PlyF307, while PlyPa91 and PlyPa96 were more distantly related.

### Example 1.3 - Cloning of additional P. aeruginosa lysins homologues to PlyPa02

Examination of the phylogenetic tree revealed a large group of lysins with close homology to PlyPa02. We further explored this group for lysins with improved killing activity. We produced lysins PlyPa03, PlyPa09, PlyPa19, PlyPa21, PlyPa29 in a modified pET21-based plasmid. The lysins were purified and 3C-cleaved, and their killing activity against *P. aeruginosa* strain PA01 was determined as described above (Fig. 1B). These results demonstrated a substantial killing activity for all lysins, with a slight advantage for PlyPa03. Based on the results, PlyPa01, PlyPa03, PlyPa91, and PlyPa96 were further analyzed.

### Example 1.4 - Activity of lysins against log-phase and stationary bacteria

We next compared the activity of PlyPa01, PlyPa03, PlyPa91, and PlyPa96 against log-phase and stationary (grown overnight) *P. aeruginosa* cells (Fig. 2). In all cases, stationary bacteria were less susceptible to killing compared to log-phase cells. However, while the activity of PlyPa01 and PlyPa96 was almost completely eliminated when used against stationary bacteria, PlyPa03 and PlyPa91 retained substantial killing activity against stationary cells.

### Example 1.5 - Killing activity of lysins against clinical isolates of P. aeruginosa and other Gram-negative pathogens

We next tested the killing activity of PlyPa01, PlyPa03, PlyPa91, and PlyPa96 against clinical isolates of *P. aeruginosa* (Fig. 3A). Following 1 h incubation, all four enzymes reduced the colony count of most strains to below detection level. For AR463, a lower respiratory tract isolate, and AR472, a urinary tract infection isolate, the reduction in viable bacteria ranged between 1-4 logs, and further reduction or complete killing was achieved using a higher lysin concentration (Fig. 16). PlyPa03 and PlyPa91 had good killing activity against most *Klebsiella and Enterobacter* strains tested, resulting in 5-log kill in most cases, while PlyPa01 and PlyPa96 displayed only weak to moderate killing activity (Fig. 3B). PlyPa03 displayed relatively weak activity against *E. coli, S. flexneri,* and *C. freundii,* but PlyPa91 was active against these species demonstrating a broader activity range (Fig. 3C). All enzymes had good activity against *A. baumannii* and *S. sonnei,* but only moderate to weak activity against *Salmonella* spp. and *P. mirabilis.* The enzymes had little to no activity against *S. marcescens* and the Gram-positive bacteria *S. aureus* and *B. anthracis.* These results indicate that despite the relatively broad range of these lysins, some level of specie specificity does exist. Based on these results we chose to proceed with PlyPa03 and PlyPa91 in further experiments.

### Example 1.6 - Time kill curve

To evaluate the relative killing activity of PlyPa03 and PlyPa91 over time, we incubated *P. aeruginosa* PA01 cells with the two lysins for time periods ranging from one minute to two hours using 100 µg/ml each (Fig. 4). PlyPa03 rapidly killed *P. aeruginosa,* resulting in >2-log kill after one minute, and reduction to below detection level after 5 minutes. PlyPa91 had a somewhat slower killing kinetics, resulting in 1-log kill after one minute, >2-log kill after 5 minutes, and reduction to below detection level after 20 minutes.

### Example 1.7 - Biochemical characterization of lysins

We next characterized the effect of pH, salt, and urea on the activity of PlyPa03 and PlyPa91. To determine the relative activity of the lysins in various pH conditions, *P*. *aeruginosa* log-phase cells were incubated with each of the lysins in buffer conditions ranging from pH 4.0 to 11.0 (Fig. 5). Incubation of bacteria at pH 4.0 and pH 11.0 resulted in a dramatic reduction in viability even in the absence of lysins, preventing evaluation of lysin activity at these pH conditions (a moderate reduction in viability was also seen in pH 10.0). Both PlyPa03 and PlyPa91 effectively killed *P. aeruginosa* in all pH conditions between 5.0 and 10.0. We further explored more subtle differences in activity at pH 6.0, 7.0, 8.0, and 9.0, by performing the experiments at various lysin concentrations (Fig. 17). Only slight differences in activity were observed among the different pH conditions, with PlyPa03 showing somewhat better activity at pH 6.0 and 7.0 compared to pH 8.0 and 9.0, and PlyPa91 showing somewhat better activity at pH 6.0 and 9.0, compared to pH 7.0 and 8.0, (Fig. 17).

We next evaluated the effect of salt on the activity of PlyPa03 and PlyPa91 (Fig. 6A). In control samples bacterial viability remained relatively constant up to 300 mM NaCl, but was slightly reduced at 500 mM NaCl, and substantially reduced at 1 M NaCl (preventing reliable estimation of lysin activity at this concentration). PlyPa03 remained active in NaCl concentrations as high as 500 mM, however the activity of PlyPa91 was substantially inhibited at 500 mM NaCl.

We also evaluated the activity of PlyPa03 and PlyPa91 in urea. In control samples no reduction in bacterial viability was seen at urea concentrations of up to 1 M, however very few viable bacteria were recovered following incubation in 2 M urea, preventing reliable evaluation of lysin activity at this concentration. Both lysins were fully active in all urea concentrations tested (Fig. 6B).

### Example 1.8 - Lysin activity in the presence of human serum

Next we tested the activity of the lysins against *P. aeruginosa* in the presence of human serum (Fig. 7). Even a minute amount of serum (1%) completely inhibited the killing activity of PlyPa03. PlyPa91 retained some activity at low serum concentrations, however it too was completely inhibited at 8% serum. As such, these lysins may not be ideal for systemic use, and would be better suited for topical applications. Nevertheless, PlyPa91 may be a better choice in topical environments where a certain amount of serum components may be expected.

### Example 1.9 - Activity of the lysins in the presence of lung surfactants

*P. aeruginosa* is among the most common causes for nosocomial pneumonia, an infection with a mortality rate as high as 30% (Williams, Dehnbostel et al. 2010). Lung surfactants are important components of the alveolar mucosa, and are critical for the maintenance of proper surface tension in the alveoli (Clements 1997). Survanta is a concentrated mixture of bovine lung surfactants and artificial surfactants, and as such could be used to approximate the effect of lung surfactant on lysin activity. PlyPa03 and PlyPa91 were fully active against *P. aeruginosa* in the presence of all Survanta concentrations tested, up to 25% (Fig. 8A). We then tested the effect of bronchoalveolar lavage (BAL) on lysin activity (Fig. 8B). *P. aeruginosa* cells were incubated with PlyPa03 or PlyPa91 in the presence of different concentration of BAL, freshly obtained as a discarded sample. PlyPa03 was active in 6.2% BAL, had a reduced activity in 12.5% BAL and was completely inhibited in 25% BAL. PlyPa91 on the other hand retained its activity up to the highest concentration of BAL tested (50%). In considering the lower sensitivity of PlyPa91 to serum, it is possible that some of the serum components are present in BAL at lower concentration, and that as a result PlyPa91 is more active in BAL.

### Example 1.10 - Biofilm

Biofilms are communities of bacteria encased in extracellular matrix that protects them from environmental conditions, including antibiotics, anti-microbioal peptides, and host response. Biofilms play an important role in many types of *P. aeruginosa* infection and are ubiquitous in the lungs of CF patients. To test the effect of PlyPa03 and PlyPa91 on *P. aeruginosa* biofilm we used the MBEC Biofilm Inoculator 96-well plate system. Biofilms were grown for 24 h on the 96-peg lid, washed twice, and treated with different concentrations of PlyPa03, PlyPa91, or buffer control for 2 h at 37°C. The pegs were washed, and surviving bacteria were recovered by sonication in 200 µl/well PBS. Quantification of surviving bacteria was done by serial dilution and plating. For PlyPa03 biofilm was completely eliminated at all concentrations tested, down to 0.375 mg/ml. Treatment with PlyPa91 resulted in >1-log CFU drop at 0.375 mg/ml, >2-log CFU drop at 0.75 mg/ml, and complete elimination of the biofilm at 1.5 mg/ml (Fig. 9). Thus, while both enzymes were effective in the elimination of *P. aeruginosa* biofilm, PlyPa03 performed substantially better.

### Example 1.11 - PlyPa03 protects mice in a skin infection model

We next tested the efficacy of PlyPa03 in a mouse model of *P. aeruginosa* skin infection. Mice were shaved, and the top layers of the epidermis were removed by tape-stripping 15-20 times. *P. aeruginosa* cells were applied to the skin and allowed to establish infection for 24 h. The infected skin was treated with 200 µg or 300 µg PlyPa03, or buffer control. Three hours later, the mice were euthanized, the infected skin was excised and homogenized, and the bacterial burden was evaluated by serial dilution and plating. The use of PlyPa03 resulted in a dose-dependent reduction in the *P. aeruginosa,* with the 300 µg dose leading to >2-log mean reduction in bacterial load (Fig. 10A). In a follow-up experiment we repeated the 300 µg dose treatment with PlyPa03, and included an additional group of mice treated with 100 µg PlyPa91 (production of a large quantity of highly concentrated PlyPa91 was more difficult compared to PlyPa03). In this experiment treatment with PlyPa03 again resulted in >2-log mean reduction in bacterial load, while 100 µg PlyPa91 resulted in 1-log reduction in bacterial counts (Fig. 10B). Given that reduction in bacterial counts was reproducible and dose dependent, it is expected that higher doses and multiple repeat doses could be used in the clinic, leading to increased efficacy.

### Example 1.12 - PlyPa91 protects mice in a lung infection model

Female C57BL/6 mice were infected by intranasal application of 2 × 50 µl of 10⁸ CFU/ml log-phase *P. aeruginosa* PA01 to establish lung infection. The mice were treated at three and six hours post infection with 50 µl of 1.8 mg/ml PlyPa91 in PBS or PBS alone by two intranasal installations or by one intranasal and one intratracheal installation. Survival of the mice was monitored daily for 10 days. The majority of the mice in the control group died within the first 24 h, and remaining mice died by the 48 h following infection (results from the two PBS treatment regiments were similar, and were combined into a single control group). Mice treated with PlyPa91 in two intranasal applications displayed a significant delay in death, with 20% of the mice surviving at day 10. Mice treated by a one intranasal and one intratracheal installation displayed a significant reduction in death rate, with 70% of the mice surviving at the day 10 (Fig. 10C). Thus, PlyPa91 displayed significant protection of the mice in this model, and the rout of delivery appears to play a critical role in ensuring the efficacy of treatment.

### Example 1.13 - Materials and Methods used to produce the results of Example 1.

### Bacterial strains and growth conditions

Table 2 describes bacterial strains used in this study and their source. *Pseudomonas* clinical isolates were obtained from the Hospital for Special Surgery in New York provided by Lars Westblade, or from NYU hospital. Gram-negative bacteria were cultured in lysogeny broth (LB, EMD Millipore), and Gram-positive bacteria were grown in Mueller Hinton broth (Difco) at 37°C, 200 rpm.

### Gene synthesis and cloning.

To facilitate analysis of *Pseudomonas* lysins, pAR553, a derivate of pBAD24 containing a new MCS (EcoRI - SalI - NotI - KpnI - XbaI - PstI) was constructed by aligning primers 629_5_pBAD_MCS (5'-aattcgtcgacggggcggccgcggtacctctagactgcag) (SEQ ID NO:24):, and 630_3_pBAD_MCS (5'-gtctagaggtaccgcggccgccccgtcgacg) (SEQ ID NO:25), and inserting the resulting double-stranded DNA into the EcoRI and PstI sites of pBAD24 (Guzman, Belin et al. 1995). *Pseudomonas* lysins were identified in the NCBI database through BLAST search using the *Acinetobacter* lysin PlyF307 as query, yielding over 100 hits. All hits were aligned using the Lasergene MegAlign Pro software, with the MUSCLE algorithm. A candidate was selected from each group (see Table 4 for protein identifiers). Nucleotide sequences for selected lysins were designed with an upstream SalI and a downstream NotI restriction sites, and were synthesized by Genewiz. Creation of plasmids for the initial screen was done by inserting the lysin sequence into the SalI and NotI sites of pAR553. Creation of a 3C-cleavable hexahistidine-tagged versions of the lysins was done by inserting the lysin sequence into the SalI and NotI sites of a modified pET21 vector.

### Purification of phage lysins

An overnight culture of *E. coli* BL21 containing a lysin cloned into a modified pET21a vector was diluted 1:100 into 1 L of LB medium containing ampicillin, and placed in an environmental shaker. Upon reaching OD₆₀₀ 0.5, the expression of the lysin was induced with 0.2 mM IPTG for 4 h at 37°C, and the cells were then shaken overnight at 4°C. The cells were harvested and resuspended in 40 ml MCAC buffer (30 mM Tris pH 7.4, 0.5 M NaCl, 10% glycerol, 1 mM DTT), and homogenized. Cell debris was removed by centrifugation, and the supernatant was filtered through a 0.22-µm filter (Millipore). The cleared lysate was loaded on a NiNTA column equilibrated with MCAC buffer, followed by washes with MCAC containing 20 mM imidazole and elution with MCAC containing 150 mM imidazole. The eluted fraction was supplemented with 10× 3C buffer for a final concentration 150 mM NaCl, 50 mM tris pH 7.6, 10 mM EDTA, 1 mM DTT, and 50 µl of 3C protease were added per 1 mg of purified protein. The mix was incubated overnight at 4°C, placed in a dialysis bag with a 3 kDa cutoff, and dialyzed for 24 h against PBS with 3 buffer changes. The protein was then concentrated using an amicon ultrafiltration device, fitted with a 3-kDa molecular weight cutoff membrane, and the final concentration was determined using a ND-1000 spectrophotometer (Nanodrop), according to absorbance at 280 nm.

### Preparation of autoclaved P. aeruginosa agarose for overlay assays

*P. aeruginosa* strain PA01 was grown overnight in 6 L of LB medium, harvested, and suspended in 3 L PBS. The cells were aliquoted into bottles containing agarose to a final concentration of 0.7%, autoclaved, and stored at 4°C until use.

### Overlay assays

*E. coli* strains containing a lysin gene in pAR553 (pBAD24-based) were streaked on LB + ampicillin 15 cm glass plates containing 0.2% arabinose (to induce protein expression) overnight at 37°C. The plates were exposed to chloroform vapor for 5 minutes to permeabilize the cells. Then, soft agar containing autoclaved *P. aeruginosa* cells at 50°C was poured over the plates, covering the cells. The plates were incubated at 37°C and examined for the presence of clearing zones following 1, 2, 5, and 16 hours.

To test activity of the lysins in crude lysate, *E. coli* strains containing the gene in pAR553 were diluted 1:100 from an overnight culture into 400 ml LB + ampicillin and grown at 37°C with shaking at 200 RPM. Once the cultures reached OD₆₀₀ 0.5, arabinose was added to a final concentration of 0.2% to induce expression of the lysin. The cells were incubated for 4 h at 37°C, and placed at 4°C with gentle agitation overnight. Cells were harvested, suspended in 40 ml PBS, and homogenized. Cell debris was removed by centrifugation, and the supernatant was filtered through a 0.22-µm filter (Millipore). Varying amount of the cleared lysate was applied to a 15 cm plate containing autoclaved *P*. *aeruginosa* agarose. Observations for the presence of clearing zones were done following 1, 2, 5, and 16 hours.

### Kill assays

An overnight culture of the test bacteria was diluted 1:50 into fresh LB medium and grown to OD₆₀₀ 0.5. The cells were harvested, washed, and suspended in 30 mM HEPES buffer pH 7.4 to a final concentration of about 10⁶ cells/ml (unless otherwise noted). In a U-bottomed 96-well plate, each lysin was diluted to the desired final concentration in 50 µl 30 mM HEPES buffer, and then 50 µl of the test bacteria were added to each well. The plate was incubated for 1 h at 37°C with shaking at 200 RPM. The content of each well was then serially diluted 10-fold and streaked on LB plates to quantify viable bacteria. Mueller Hinton agar plates were used in experiments with Gram-positive bacteria.

In time kill curves, following incubation assay contents were diluted 1:1 in 5% BBL Beef Extract (BD) to stop the reaction, and were immediately diluted and plated. Assays evaluating the effect of pH were done by adding 25 µl of 100 mM of the following buffers to wells of a 96-well plate (final concentration 25 mM): pH 4.0 and 5.0 - acetate buffer; pH 6.0 - MES buffer; pH 7.0 and 8.0 - HEPES buffer; pH 9.0 - CHES buffer; pH 10.0 and 11.0 - CAPS buffer. Bacteria and lysins were diluted in deionized water rather than buffer as not to affect the final pH of the reactions. Assays evaluating the effect of salt, urea, and EDTA were carried out in 30 mM HEPES Buffer pH 7.4, 100 µg/ml lysins. Evaluation of the effect of serum was done in 30 mM HEPES Buffer pH 7.4, 100 µg/ml lysins, using serially diluted pooled human serum from male subjects, AB blood type (Sigma). Experiments in Survanta (beractant, Abbvie) were carried out in 30 mM HEPES Buffer pH 7.4, and 100 µg/ml of lysins.

### Biofilm assays

An overnight culture of *P*. *aeruginosa* PA01 was diluted 1:1000 in TSB containing 0.2% glucose. The diluted bacteria were added to an MBEC Biofilm Inoculator 96-well plate (Innovotech #9111) at 100 µl/well and placed in a plastic bag with a wet paper towel to maintain humidity. Biofilm was grown at 37°C for 24 h at 65 RPM. The 96 peg lid which contained established biofilm was removed and washed twice using 96-well plates with 200 µl/well PBS. The washed biofilm was then transferred to a 96-well plate containing 200 µl/well of the lysins or controls and placed in a 37°C shaker at 65 RPM for 2 h. The biofilms were then washed with PBS as described above and transferred to a 96-well plate containing 200 µl/well PBS for recovery by water bath sonication for 30 min. Quantification of surviving cells was done by serial dilutions and plating.

### Mouse skin infection model

The skin infection model was based on Pastagia et al. (Pastagia, Euler et al. 2011). Female CD1 mice, 6-8 weeks old (Charles River Laboratories, Wilmington, MA), were anesthetized by an IP injection of ketamine (1.2 mg/animal) and xylazine (0.25 mg/animal). The back of the mice was shaven with an electric razor and treated with Nair to remove remaining hair. Then, an area of 2-cm² was tape-stripped 15-20 times using autoclave tape (using a fresh piece of tape each time); two experimental areas were prepared for each mouse, and these were treated in a similar manner (treatment or control) to prevent cross contamination. The tape stripped areas were then sterilized using alcohol wipes, allowed to dry for a few minutes, and then treated with 10 µl log-phase *P. aeruginosa* PA01 at a concentration of 5×10⁶/ml. Infection was allowed to establish for 24 hours, and the mice were then treated with two sequential 25 µl doses of lysin in CAPS buffered saline pH 6.0 or buffer control. Three hours following treatment, mice were euthanized, and the wound area was excised. Each skin sample was homogenized in 500 µl PBS using a stomacher 80 Biomaster machine (Seward Ltd., United Kingdom). The homogenate was serially diluted and plated on LB plates supplemented by ampicillin as a selective agent to prevent growth of normal skin flora (*P. aeruginosa* is resistant to ampicillin), in order to calculate the *P. aeruginosa* CFUs in the skin sample.

### Mouse lung infection model

Female C57BL/6 mice, 9-10 weeks old (Charles River Laboratories, Wilmington, MA), were anesthetized using isoflurane. Lung infection was established by intranasal application of 2 × 50 µl of 10⁸ CFU/ml log-phase *P. aeruginosa* PA01. At three and six hours after infection, mice were anesthetized with isoflurane and treated with 50 µl of 1.8 mg/ml PlyPa91 in PBS or PBS alone by intranasal installation. The mice were treated at three and six hours post infection with 50 µl of 1.8 mg/ml PlyPa91 in PBS or PBS alone by two intranasal installations or by one intranasal and one intratracheal installation. Survival of the mice was monitored daily for 10 days. The data were statistically analyzed using Kaplan-Meier survival curves with standard errors, 95% confidence intervals, and significance levels (log rank/Mantel-Cox test) calculated using the Prism 7 computer program (GraphPad Software, La Jolla, CA).

It will be recognized from the results described herein that in for this Example, the large number of available *P. aeruginosa* sequenced genomes were used in part to identify lysins with innate killing activity against *P. aeruginosa.* We used the sequence of the *A. baumannii* PlyF307 (Lood, Winer et al. 2015) as a starting point for a BLAST analysis of *Pseudomonas* genomes, yielding over 100 hits. Through phylogenetic analysis and successive rounds of lysin expression and characterization, two lysins of this Example exhibit were shown to exhibit substantial killing activity against *P*. *aeruginosa.* These lysins, PlyPa03 and PlyPa91, were active against log phase and stationary bacteria, and were able to kill a wide range of Gram-negative organisms including clinical isolates of *P. aeruginosa, A. baumannii, K. pneumonia,* and *E. cloacae.* The lysins were active in a broad pH range, high salt and urea concentrations, and in the presence of lung surfactants (Survanta). Although neither of the two enzymes was active in human serum, PlyPa91 retained activity in low serum concentrations, indicating that it may be better of the enzymes in the treatment of topical or mucosal infections, where a low level of serum components may be present. Nevertheless, mouse models of skin infection demonstrate that PlyPa03 is still useful in the treatment of topical infection, leading to 2.5-log reduction in bacteria following a single treatment.

Prior to the present disclosure, *P. aeruginosa* colonization and infection of topical and mucosal environments remained an important area of unmet need. *P. aeruginosa* is the second most commonly isolated organisms from patients with ventilator-associated pneumonia (VAP) (Hidron, Edwards et al. 2008), an infection that has a mortality rate as high as 30% (Williams, Dehnbostel et al. 2010). Certain populations are highly prone to intermittent or chronic colonization of their lungs with *P. aeruginosa* regardless of intubation, including patients with cystic fibrosis (CF), chronic obstructive pulmonary disease (COPD), neutropenia due to cancer chemotherapy, or immunosuppression due to organ transplant (Williams, Dehnbostel et al. 2010). CF patients are almost invariably colonized with *P*. *aeruginosa,* and such infections can last 20 years or more (Bragonzi, Paroni et al. 2009) (Smith, Buckley et al. 2006) (Williams, Dehnbostel et al. 2010). In patients suffering from COPD, the incidence of infection ranges between 4-15%, with many patients developing chronic infections (Williams, Dehnbostel et al. 2010). In burn wound patients, the compromised state of the skin barrier lead to a high risk of infection with *P. aeruginosa,* as this organism is ubiquitous in the environment and on fomites in hospital wards. Infection with *P. aeruginosa* results in a worsen prognosis and a risk of sepsis. Acute otitis externa (swimmers ear) is an infection of the ear canal that affects 4 in 1000 people per year. The infection is predominantly of bacterial etiology, and 50% of cases are due to *P. aeruginosa* (Osguthorpe and Nielsen 2006). Ulcerative keratitis is a bacterial infection causing an inflammatory response of the cornea, often associated with injury or trauma to the cornea or the use extended-wear soft contact lenses (Galentine, Cohen et al. 1984) (Weissman, Mondino et al. 1984). Thus, in embodiments, a method of this disclosure is used to affect growth of bacteria in a wound. In embodiments, the wound comprises a burn, such as a burn that comprises tissue damage induced by contact with heated objects and/or surfaces, or light, or chemicals. In embodiments, the wound is caused by medical techniques such as surgical interventions wherein the skin, other tissue or an organ is cut or pierced or avulsed, or other non-medical wounds which cause trauma by any means. In an embodiment, the infection is a catheter-associated urinary tract infection, or is associated with intubation and/or a device used for intubation, and/or for delivery of anesthesia. In other embodiments, the bacteria are present in a biofilm. In embodiments, the individual has an infection of blood.

An effective lysin, such as those described herein in Table 1, Example 1, could be greatly beneficial in the treatment of multidrug resistant *P. aeruginosa* infections in these environments. Of the two enzymes described in this particular Example, and without intending to be bound by any particular theory, it is presently considered that PlyPa91 is a better option for use in mucosal environments due to its higher resistance to inhibition by serum components, some of which may be present in mucosal environments. Lung surfactants do not appear to inhibit the activity of the two enzymes described in this Example, further supporting the use of these enzymes in the lung environment. As described above, delivery of lysins to the lung alveoli could be performed by aerosol inhalation as done with antibiotics (Brown, Kruse et al. 1990) (Luyt, Clavel et al. 2009) (Michalopoulos, Fotakis et al. 2008) (McCoy, Quittner et al. 2008), reviewed by (Falagas, Agrafiotis et al. 2008). A proof of concept study has shown that aerosolized Cpl-1 lysin if effective in the treatment of pneumococcal pneumonia in a murine model of infection (Doehn, Fischer et al. 2013). It is therefore reasonable that PlyPa91 could be used in a similar manner in the treatment of *P. aeruginosa* lung infections. For certain topical applications such as burn wounds, otitis externa, and ulcerative keratitis, lysins could be formulated a topical solution or an ointment (Pastagia, Euler et al. 2011), as further described above. Lysins could also be used preventatively, for example in the dressing of burn wounds or in contact lens solution. Thus, use in treatment and/or prophylaxis of ocular infections is included in the disclosure. Further, lysins have several advantages when compared with traditional antibiotics. Lysins kill bacteria faster than antibiotics do, and resistance to lysins is a rare event due to the conserved nature of their peptidoglycan target. Resistance was not observed for lysins targeting Gram-positive bacteria (Loeffler, Nelson et al. 2001, Schuch, Nelson et al. 2002), or for the artilysin Art-175 when tested against *P*. *aeruginosa* and *A. baumannii* (Briers, Walmagh et al. 2014) (Defraine, Schuermans et al. 2016).

Thus, certain polypeptides described herein are highly active against various *P*. *aeruginosa* clinical isolates, *A. baumannii,* and several members of the Enterobacteriaceae including *Klebsiella* and *Enterobacter* species. The enzymes were active in a broad pH range and high concentrations of salt, urea, and lung surfactants. Although the sensitivity of the enzymes to serum make them not preferable for systemic use, and again without intending to be bound by any particular theory, PlyPa91 in particular is expected to be effective for the treatment of *P. aeruginosa* infections in various topical and mucosal environments.

**Table 2.**

| **Lysin** | **Colony overlay** | **Induced lysate** |
|---|---|---|
| PlyPa01 | + | + |
| PlyPa02 | + | + |
| PlyPa40 | + | + |
| PlyPa49 | + | + |
| PlyPa58 | - | + |
| PlyPa64 | + | + |
| PlyPa78 | - | - |
| PlyPa80 | - | - |
| PlyPa91 | + | + |
| PlyPa92 | - | - |
| PlyPa96 | + | + |

For Table 2, for colony overlays, *E. coli* colonies expressing various lysins were permeabilized and overlaid with soft agar containing autoclaved *P. aeruginosa.* Alternatively, induced lysates were produced for the various lysins and spotted over a layer of agar containing autoclaved *P. aeruginosa* cells. The presence of a clearing zone is denoted by a plus sign (+).

**Table 3 - Strains used in this Example**

| Organism | Source | Strain identifier |
|---|---|---|
| *A. baumannii,* ATCC 17978 | ATCC | 391 |
| *A. baumannii,* ATCC BAA-1791 | ATCC | 392 |
| *B. anthracis,* Δ Stern | (Schuch, Nelson et al. 2002) | 34 |
| *C. freundii,* ATCC 8090 | ATCC | 405 |
| *E. aerogenes,* NR-48555 (CRE) | BEI | 507 |
| *E. Cloacae,* NR-50391 | BEI | 504 |
| *E. Cloacae,* NR-50392 | BEI | 505 |
| *E. Cloacae,* NR-50393 | BEI | 506 |
| *E. coli,* DH5α | Invitrogen | Coli 1 |
| *E. coli,* AR531 | NYU Hospital (UT1) | 531 |
| *K. pneumoniae,* ATCC700603 | ATCC | 393 |
| *K. pneumoniae,* ATCC10031 | ATCC | 394 |
| *K. pneumoniae,* ATCC700603 | ATCC | 530 |
| *K. pneumoniae,* NR-15410 (bla_{KPC}) | BEI | 490 |
| *K. pneumoniae,* NR-15411 (bla_{KPC}) | BEI | 491 |
| *K. pneumoniae,* NR-41923 | BEI (Urine) | 493 |
| *K. pneumoniae,* NR-44349 | BEI (Sepsis) | 496 |
| *P. aeruginosa,* PA01 | ATCC | 437 |
| *P. aeruginosa,* AR443 | Cornell Hospital | 443 |
| *P. aeruginosa,* AR444 | Cornell Hospital | 444 |
| *P. aeruginosa,* AR461 | NYU Hospital (LRT) | 461 |
| *P. aeruginosa,* AR463 | NYU Hospital (LRT) | 463 |
| *P. aeruginosa,* AR465 | NYU Hospital (LRT) | 465 |
| *P. aeruginosa,* AR468 | NYU Hospital (wound) | 468 |
| *P. aeruginosa,* AR469 | NYU Hospital (wound) | 469 |
| *P. aeruginosa,* AR470 | NYU Hospital (stool) | 470 |
| *P. aeruginosa,* AR471 | NYU Hospital (UTI) | 471 |
| *P. aeruginosa,* AR472 | NYU Hospital (UTI) | 472 |
| *P. aeruginosa,* AR474 | NYU Hospital (UTI) | 474 |
| *P. mirabilis,* AR397 | Hunter College Collection | 397 |
| Salmonella spp. Serogroup D AR396 | Hunter College Collection | 396 |
| *S*. *marcescens,* AR401 | Hunter College Collection | 401 |
| *S. flexneri,* ATCC 12022 | ATCC | 404 |
| *S. sonnei,* ATCC 25931 | ATCC | 395 |
| *S*. *aureus,* Newman | (Daniel, Euler et al. 2010) | 220 |

**Table 4**

| Protein identifiers | |
|---|---|
| **Lysin** | **Protein identifier** |
| PlyPa01 | WP_058157505 |
| PlyPa02 | WP_073667504 |
| PlyPa03 | WP_070344501 |
| PlyPa09 | WP_042930029 |
| PlyPa19 | WP_034013816 |
| PlyPa21 | WP_042853300 |
| PlyPa29 | WP_058158945 |
| PlyPa40 | WP_058171189 |
| PlyPa49 | WP_058355500 |
| PlyPa58 | WP_058182687 |
| PlyPa64 | WP_033973815 |
| PlyPa78 | WP_034067975 |
| PlyPa80 | WP_057386760 |
| PlyPa91 | CRR10611 |
| PlyPa92 | WP_052160556 |
| PlyPa96 | WP_019681133 |

### Example 2 (Reference only)

In an approach that differs from Example 1, we isolated bacteriophage lysins from native bacteriophages. Modification of these lysins display increased activity in serum, relative to unmodified lysins.

We amplified the genes encoding the lysins of phages NP1 and NP3 from phage genomic DNA and expressed them as hexahistidine-tagged 3C-cleavable products. The lysin from phage NP1 was termed PlyPa101, and the lysin from phage NP3 was termed PlyPa102. Through BLAST searches for *P. aeruginosa* genomes and screening we identified PlyPa103 - a homologue of PlyPa101 - and this gene was similarly expressed as a hexahistidine fusion protein that is cleavable by 3C protease. The proteins were expressed and purified as described above.

The purified proteins were tested for their ability to Kill *P. aeruginosa* and *Klebsiella* spp. (Fig. 18). PlyPa101 and PlyPa103 displayed killing for both species, while PlyPa102 did not displayed killing activity. Based on these results PlyPa101 and PlyPa103 were further characterized.

Various concentrations of PlyPa101 and PlyPa103 were tested against *E*. *coli, K. pneumoniae, C. freundii, E. aerogenes,* and *Acinetobacter baumannii* (Fig. 19). Both enzymes were active against all the organisms tested, with *A. baumannii* being the most sensitive to the activity of the lysins, followed by *E. aerogenes,* and the other members of the *Enterobacteriaceae.*

We further analyzed the activity range of the two lysins against a large range of clinical isolates of *P. aeruginosa, K. pneumoniae, E. cloacae,* and various other bacteria (Fig. 20). Both enzymes were active against most strains of *P. aeruginosa,* with slight advantage for PlyPa103. While PlyPa101 was not very active against *Klebsiella,* Enterobacter and other Enterobacteriaceae, PlyPa103 led to several-log reduction in many of the strains. Both enzymes were active against *A. baumannii* but neither enzymes were active *against S. flexneri, S. marcescens, S. aureus, and B. anthracis.*

We next tested the effect of salt on the activity of PlyPa101 and PlyPa103 (Fig. 21). The two lysins were active in all salt concentrations tested, however when cells were incubated in the presence of 500 mM NaCl substantial death occurred even in the absence of lysin, preventing accurate estimate of lysin activity at this concentration.

Next, we tested the effect of urea on the activity of the two lysins (Fig. 22). Both PlyPa101 and PlyPa103 were fully active in all urea concentrations tested up to 500 mM. Next, we tested the activity of the enzymes in the presence of 7.5% of the lung surfactant mixture Survanta (Fig. 23). Both enzymes were fully active in this Survanta concentration. Next, we tested the activity of the lysins in the presence of human serum (pooled human serum from blood type AB donors) (Fig. 24). Both enzymes were inhibited by human serum, however PlyPa103 was active in serum concentrations of up to 3%.

In order to improve the activity of PlyPa101 in human serum we created 6 fusion proteins with various antimicrobial peptides (AMPs), described in Table 1. These were produced as hexahistidine tag and GST tag fusion proteins cleavable by 3C protease as described above. In some embodiments the AMP was separated from the lysin by a flexible linker. For example in SEQ IDs 19-24 in Table 1 a flexible linker comprising of amino acids GGGSGGGSGGG (SEQ ID NO:28) was included between Lysin PlyPa101 and the AMP sequence. Inclusion of a flexible linkers and their sequences are known in the art. For some examples of suitable methods links, see, for example, of varying sizes is common practice for people skilled in the art as has been reviewed (Chen et al., 2013; Adv Drug Deliv Rev. 2013 Oct 15; 65(10): 1357-1369. In some embodiments a flexible or rigid linkers may be added or omitted. Representative and non-limiting examples of linker sequences are as shown in Table 1, wherein the linker sequences are italicized.

We then compared the activity of the different fusion proteins in the presence of human serum (Fig. 25). All fusion proteins performed better compared to the parental (native) protein. In particular, and without intending to be bound by any particular theory, PlyPa101-AMP1 (LL-37) and PlyPa101-AMPS (RP-1) had the best activity, resulting in 4-fold increase in resistance to serum inhibition.

The invention is defined by the appended claims.
The following references are cited in this application or patent. This reference listing is not an indication that any of the references are material to patentability
Arima, H., H. R. Ibrahim, T. Kinoshita and A. Kato (1997). "Bactericidal action of lysozymes attached with various sizes of hydrophobic peptides to the C-terminal using genetic modification." FEBS Lett 415(1): 114-118.
Bilton, D. (2008). "Update on non-cystic fibrosis bronchiectasis." Curr Opin Pulm Med 14(6): 595-599.
Bjarnsholt, T., P. O. Jensen, M. J. Fiandaca, J. Pedersen, C. R. Hansen, C. B. Andersen, T. Pressler, M. Givskov and N. Hoiby (2009). "Pseudomonas aeruginosa biofilms in the respiratory tract of cystic fibrosis patients." Pediatr Pulmonol 44(6): 547-558.
Bragonzi, A., M. Paroni, A. Nonis, N. Cramer, S. Montanari, J. Rejman, C. Di Serio, G. Doring and B. Tummler (2009). "Pseudomonas aeruginosa microevolution during cystic fibrosis lung infection establishes clones with adapted virulence." Am J Respir Crit Care Med 180(2): 138-145.
Branda, S. S., S. Vik, L. Friedman and R. Kolter (2005). "Biofilms: the matrix revisited." Trends Microbiol 13(1): 20-26.
Breidenstein, E. B., C. de la Fuente-Nunez and R. E. Hancock (2011). "Pseudomonas aeruginosa: all roads lead to resistance." Trends Microbiol 19(8): 419-426.
Briers, Y., G. Volckaert, A. Cornelissen, S. Lagaert, C. W. Michiels, K. Hertveldt and R. Lavigne (2007). "Muralytic activity and modular structure of the endolysins of Pseudomonas aeruginosa bacteriophages phiKZ and EL." Mol Microbiol 65(5): 1334-1344.
Briers, Y., M. Walmagh, B. Grymonprez, M. Biebl, J. P. Pirnay, V. Defraine, J. Michiels, W. Cenens, A. Aertsen, S. Miller and R. Lavigne (2014). "Art-175 is a highly efficient antibacterial against multidrug-resistant strains and persisters of Pseudomonas aeruginosa." Antimicrob Agents Chemother 58(7): 3774-3784.
Briers, Y., M. Walmagh and R. Lavigne (2011). "Use of bacteriophage endolysin EL188 and outer membrane permeabilizers against Pseudomonas aeruginosa." J Appl Microbiol 110(3): 778-785.
Briers, Y., M. Walmagh, V. Van Puyenbroeck, A. Cornelissen, W. Cenens, A. Aertsen, H. Oliveira, J. Azeredo, G. Verween, J. P. Pirnay, S. Miller, G. Volckaert and R. Lavigne (2014). "Engineered endolysin-based "Artilysins" to combat multidrug-resistant gram-negative pathogens." MBio 5(4): e01379-01314.
Brown, R. B., J. A. Kruse, G. W. Counts, J. A. Russell, N. V. Christou and M. L. Sands (1990). "Double-blind study of endotracheal tobramycin in the treatment of gram-negative bacterial pneumonia. The Endotracheal Tobramycin Study Group." Antimicrob Agents Chemother 34(2): 269-272.
Carmeli, Y., N. Troillet, A. W. Karchmer and M. H. Samore (1999). "Health and economic outcomes of antibiotic resistance in Pseudomonas aeruginosa." Arch Intern Med 159(10): 1127-1132.
Chang, H. J., P. C. Hsu, C. C. Yang, A. J. Kuo, J. H. Chia, T. L. Wu and M. H. Lee (2011). "Risk factors and outcomes of carbapenem-nonsusceptible Escherichia coli bacteremia: a matched case-control study." J Microbiol Immunol Infect 44(2): 125-130.
Chitkara, Y. K. and T. C. Feierabend (1981). "Endogenous and exogenous infection with Pseudomonas aeruginosa in a burns unit." Int Surg 66(3): 237-240.
Clements, J. A. (1997). "Lung surfactant: a personal perspective." Annu Rev Physiol 59: 1-21.
Cornaglia, G., H. Giamarellou and G. M. Rossolini (2011). "Metallo-beta-lactamases: a last frontier for beta-lactams?" Lancet Infect Dis 11(5): 381-393.
Davies, J. C. and D. Bilton (2009). "Bugs, biofilms, and resistance in cystic fibrosis." Respir Care 54(5): 628-640.
Defraine, V., J. Schuermans, B. Grymonprez, S. K. Govers, A. Aertsen, M. Fauvart, J.
Michiels, R. Lavigne and Y. Briers (2016). "Efficacy of Artilysin Art-175 against Resistant and Persistent Acinetobacter baumannii." Antimicrob Agents Chemother 60(6): 3480-3488. Delcour, A. H. (2009). "Outer membrane permeability and antibiotic resistance." Biochim Biophys Acta 1794(5): 808-816.
Dhungana, S., C. H. Taboy, D. S. Anderson, K. G. Vaughan, P. Aisen, T. A. Mietzner and A. L. Crumbliss (2003). "The influence of the synergistic anion on iron chelation by ferric binding protein, a bacterial transferrin." Proc Natl Acad Sci U S A 100(7): 3659-3664.
Diez-Martinez, R., H. D. de Paz, N. Bustamante, E. Garcia, M. Menendez and P. Garcia (2013). "Improving the lethal effect of cpl-7, a pneumococcal phage lysozyme with broad bactericidal activity, by inverting the net charge of its cell wall-binding module." Antimicrob Agents Chemother 57(11): 5355-5365.
Doehn, J. M., K. Fischer, K. Reppe, B. Gutbier, T. Tschernig, A. C. Hocke, V. A. Fischetti, J. Loffler, N. Suttorp, S. Hippenstiel and M. Witzenrath (2013). "Delivery of the endolysin Cpl-1 by inhalation rescues mice with fatal pneumococcal pneumonia." J Antimicrob Chemother 68(9): 2111-2117.
El Solh, A. A. and A. Alhajhusain (2009). "Update on the treatment of Pseudomonas aeruginosa pneumonia." J Antimicrob Chemother 64(2): 229-238.
Ernst, R. K., S. M. Moskowitz, J. C. Emerson, G. M. Kraig, K. N. Adams, M. D. Harvey, B. Ramsey, D. P. Speert, J. L. Burns and S. I. Miller (2007). "Unique lipid a modifications in Pseudomonas aeruginosa isolated from the airways of patients with cystic fibrosis." J Infect Dis 196(7): 1088-1092.
Evans, M. E., D. J. Feola and R. P. Rapp (1999). "Polymyxin B sulfate and colistin: old antibiotics for emerging multiresistant gram-negative bacteria." Ann Pharmacother 33(9): 960-967.
Evans, R. C. and C. J. Holmes (1987). "Effect of vancomycin hydrochloride on Staphylococcus epidermidis biofilm associated with silicone elastomer." Antimicrob Agents Chemother 31(6): 889-894.
Falagas, M. E., M. Agrafiotis, Z. Athanassa and Siempos, II (2008). "Administration of antibiotics via the respiratory tract as monotherapy for pneumonia." Expert Rev Anti Infect Ther 6(4): 447-452.
Falagas, M. E., I. A. Bliziotis, S. K. Kasiakou, G. Samonis, P. Athanassopoulou and A. Michalopoulos (2005). "Outcome of infections due to pandrug-resistant (PDR) Gram-negative bacteria." BMC Infect Dis 5: 24.
Falagas, M. E., M. Rizos, I. A. Bliziotis, K. Rellos, S. K. Kasiakou and A. Michalopoulos (2005). "Toxicity after prolonged (more than four weeks) administration of intravenous colistin." BMC Infect Dis 5: 1.
Falagas, M. E., G. S. Tansarli, D. E. Karageorgopoulos and K. Z. Vardakas (2014). "Deaths attributable to carbapenem-resistant Enterobacteriaceae infections." Emerg Infect Dis 20(7): 1170-1175.
Fischetti, V. A. (2010). "Bacteriophage endolysins: a novel anti-infective to control Gram-positive pathogens." Int J Med Microbiol 300(6): 357-362.
Galentine, P. G., E. J. Cohen, P. R. Laibson, C. P. Adams, R. Michaud and J. J. Arentsen (1984). "Corneal ulcers associated with contact lens wear." Arch Ophthalmol 102(6): 891-894.
Gayoso, C. M., J. Mateos, J. A. Mendez, P. Fernandez-Puente, C. Rumbo, M. Tomas, O. Martinez de Ilarduya and G. Bou (2014). "Molecular mechanisms involved in the response to desiccation stress and persistence in Acinetobacter baumannii." J Proteome Res 13(2): 460-476.
Gilmer, D. B., J. E. Schmitz, C. W. Euler and V. A. Fischetti (2013). "Novel bacteriophage lysin with broad lytic activity protects against mixed infection by Streptococcus pyogenes and methicillin-resistant Staphylococcus aureus." Antimicrob Agents Chemother 57(6): 2743-2750.
Gristina, A. G., C. D. Hobgood, L. X. Webb and Q. N. Myrvik (1987). "Adhesive colonization of biomaterials and antibiotic resistance." Biomaterials 8(6): 423-426. Guzman, L. M., D. Belin, M. J. Carson and J. Beckwith (1995). "Tight regulation, modulation, and high-level expression by vectors containing the arabinose PBAD promoter." J Bacteriol 177(14): 4121-4130.
Hancock, R. E. (1998). "Resistance mechanisms in Pseudomonas aeruginosa and other nonfermentative gram-negative bacteria." Clin Infect Dis 27 Suppl 1: S93-99.
Hidron, A. I., J. R. Edwards, J. Patel, T. C. Horan, D. M. Sievert, D. A. Pollock, S. K. Fridkin, T. National Healthcare Safety Network and F. Participating National Healthcare Safety Network (2008). "NHSN annual update: antimicrobial-resistant pathogens associated with healthcare-associated infections: annual summary of data reported to the National Healthcare Safety Network at the Centers for Disease Control and Prevention, 2006-2007." Infect Control Hosp Epidemiol 29(11): 996-1011.
Ibrahim, H. R., M. Yamada, K. Matsushita, K. Kobayashi and A. Kato (1994). "Enhanced bactericidal action of lysozyme to Escherichia coli by inserting a hydrophobic pentapeptide into its C terminus." J Biol Chem 269(7): 5059-5063.
Junn, H. J., J. Youn, K. H. Suh and S. S. Lee (2005). "Cloning and expression of Klebsiella phage K11 lysozyme gene." Protein Expr Purif 42(1): 78-84.
Kidd, T. J., K. A. Ramsay, H. Hu, G. B. Marks, C. E. Wainwright, P. T. Bye, M. R. Elkins, P. J. Robinson, B. R. Rose, J. W. Wilson, K. Grimwood, S. C. Bell and A. C. I. Group (2013). "Shared Pseudomonas aeruginosa genotypes are common in Australian cystic fibrosis centres." Eur Respir J 41(5): 1091-1100.
King, J. D., D. Kocincova, E. L. Westman and J. S. Lam (2009). "Review: Lipopolysaccharide biosynthesis in Pseudomonas aeruginosa." Innate Immun 15(5): 261-312. Knirel, Y. A., O. V. Bystrova, N. A. Kocharova, U. Zahringer and G. B. Pier (2006). "Conserved and variable structural features in the lipopolysaccharide of Pseudomonas aeruginosa." J Endotoxin Res 12(6): 324-336.
Koulenti, D., T. Lisboa, C. Brun-Buisson, W. Krueger, A. Macor, J. Sole-Violan, E. Diaz, A. Topeli, J. DeWaele, A. Cameiro, I. Martin-Loeches, A. Armaganidis, J. Rello and E.-V. C. S. Group (2009). "Spectrum of practice in the diagnosis of nosocomial pneumonia in patients requiring mechanical ventilation in European intensive care units." Crit Care Med 37(8): 2360-2368.
Kramer, B. and F. F. Tisdall (1922). "THE DISTRIBUTION OF SODIUM, POTASSIUM, CALCIUM, AND MAGNESIUM BETWEEN THE CORPUSCLES AND SERUM OF HUMAN BLOOD." Journal of Biological Chemistry 53(2): 241-252.
Lai, M. J., N. T. Lin, A. Hu, P. C. Soo, L. K. Chen, L. H. Chen and K. C. Chang (2011). "Antibacterial activity of Acinetobacter baumannii phage varphiAB2 endolysin (LysAB2) against both gram-positive and gram-negative bacteria." Appl Microbiol Biotechnol 90(2): 529-539.
Lam, J. S., V. L. Taylor, S. T. Islam, Y. Hao and D. Kocincova (2011). "Genetic and Functional Diversity of Pseudomonas aeruginosa Lipopolysaccharide." Front Microbiol 2: 118.
Larpin, Y., F. Oechslin, P. Moreillon, G. Resch, J. M. Entenza and S. Mancini (2018). "In vitro characterization of PlyE146, a novel phage lysin that targets Gram-negative bacteria." PLoS One 13(2): e0192507.
Li, J., R. L. Nation, J. D. Turnidge, R. W. Milne, K. Coulthard, C. R. Rayner and D. L. Paterson (2006). "Colistin: the re-emerging antibiotic for multidrug-resistant Gram-negative bacterial infections." Lancet Infect Dis 6(9): 589-601.
Livermore, D. M. (2002). "Multiple mechanisms of antimicrobial resistance in Pseudomonas aeruginosa: our worst nightmare?" Clin Infect Dis 34(5): 634-640.
Loeffler, J. M., D. Nelson and V. A. Fischetti (2001). "Rapid killing of Streptococcus pneumoniae with a bacteriophage cell wall hydrolase." Science 294(5549): 2170-2172. Lood, R., B. Y. Winer, A. J. Pelzek, R. Diez-Martinez, M. Thandar, C. W. Euler, R. Schuch and V. A. Fischetti (2015). "Novel phage lysin capable of killing the multidrug-resistant gram-negative bacterium Acinetobacter baumannii in a mouse bacteremia model." Antimicrob Agents Chemother 59(4): 1983-1991.
Lukacik, P., T. J. Barnard, P. W. Keller, K. S. Chaturvedi, N. Seddiki, J. W. Fairman, N. Noinaj, T. L. Kirby, J. P. Henderson, A. C. Steven, B. J. Hinnebusch and S. K. Buchanan (2012). "Structural engineering of a phage lysin that targets gram-negative pathogens." Proc Natl Acad Sci U S A 109(25): 9857-9862.
Luyt, C. E., M. Clavel, K. Guntupalli, J. Johannigman, J. I. Kennedy, C. Wood, K. Corkery, D. Gribben and J. Chastre (2009). "Pharmacokinetics and lung delivery of PDDS-aerosolized amikacin (NKTR-061) in intubated and mechanically ventilated patients with nosocomial pneumonia." Crit Care 13(6): R200.
Lyczak, J. B., C. L. Cannon and G. B. Pier (2000). "Establishment of Pseudomonas aeruginosa infection: lessons from a versatile opportunist." Microbes Infect 2(9): 1051-1060. Mah, T. F. and G. A. O'Toole (2001). "Mechanisms of biofilm resistance to antimicrobial agents." Trends Microbiol 9(1): 34-39.
McCoy, K. S., A. L. Quittner, C. M. Oermann, R. L. Gibson, G. Z. Retsch-Bogart and A. B. Montgomery (2008). "Inhaled aztreonam lysine for chronic airway Pseudomonas aeruginosa in cystic fibrosis." Am J Respir Crit Care Med 178(9): 921-928.
McPhee, J. B., M. Bains, G. Winsor, S. Lewenza, A. Kwasnicka, M. D. Brazas, F. S. Brinkman and R. E. Hancock (2006). "Contribution of the PhoP-PhoQ and PmrA-PmrB two-component regulatory systems to Mg2+-induced gene regulation in Pseudomonas aeruginosa." J Bacteriol 188(11): 3995-4006.
McPhee, J. B., S. Lewenza and R. E. Hancock (2003). "Cationic antimicrobial peptides activate a two-component regulatory system, PmrA-PmrB, that regulates resistance to polymyxin B and cationic antimicrobial peptides in Pseudomonas aeruginosa." Mol Microbiol 50(1): 205-217.
Michalopoulos, A., D. Fotakis, S. Virtzili, C. Vletsas, S. Raftopoulou, Z. Mastora and M. E. Falagas (2008). "Aerosolized colistin as adjunctive treatment of ventilator-associated pneumonia due to multidrug-resistant Gram-negative bacteria: a prospective study." Respir Med 102(3): 407-412.
Miller, A. K., M. K. Brannon, L. Stevens, H. K. Johansen, S. E. Selgrade, S. I. Miller, N. Hoiby and S. M. Moskowitz (2011). "PhoQ mutations promote lipid A modification and polymyxin resistance of Pseudomonas aeruginosa found in colistin-treated cystic fibrosis patients." Antimicrob Agents Chemother 55(12): 5761-5769.
Morinaga, Y., K. Yanagihara, S. Nakamura, K. Yamamoto, K. Izumikawa, M. Seki, H. Kakeya, Y. Yamamoto, Y. Yamada, S. Kohno and S. Kamihira (2008). "In vivo efficacy and pharmacokinetics of tomopenem (CS-023), a novel carbapenem, against Pseudomonas aeruginosa in a murine chronic respiratory tract infection model." J Antimicrob Chemother 62(6): 1326-1331.
Morita, M., Y. Tanji, Y. Orito, K. Mizoguchi, A. Soejima and H. Unno (2001). "Functional analysis of antibacterial activity of Bacillus amyloliquefaciens phage endolysin against Gram-negative bacteria." FEBS Lett 500(1-2): 56-59.
Nicas, T. I. and R. E. Hancock (1983). "Pseudomonas aeruginosa outer membrane permeability: isolation of a porin protein F-deficient mutant." J Bacteriol 153(1): 281-285. Nickel, J. C., I. Ruseska, J. B. Wright and J. W. Costerton (1985). "Tobramycin resistance of Pseudomonas aeruginosa cells growing as a biofilm on urinary catheter material." Antimicrob Agents Chemother 27(4): 619-624.
Orito, Y., M. Morita, K. Hori, H. Unno and Y. Tanji (2004). "Bacillus amyloliquefaciens phage endolysin can enhance permeability of Pseudomonas aeruginosa outer membrane and induce cell lysis." Appl Microbiol Biotechnol 65(1): 105-109.
Osguthorpe, J. D. and D. R. Nielsen (2006). "Otitis externa: Review and clinical update." Am Fam Physician 74(9): 1510-1516.
Pastagia, M., C. Euler, P. Chahales, J. Fuentes-Duculan, J. G. Krueger and V. A. Fischetti (2011). "A novel chimeric lysin shows superiority to mupirocin for skin decolonization of methicillin-resistant and -sensitive Staphylococcus aureus strains." Antimicrob Agents Chemother 55(2): 738-744.
Pastagia, M., R. Schuch, V. A. Fischetti and D. B. Huang (2013). "Lysins: the arrival of pathogen-directed anti-infectives." J Med Microbiol 62(Pt 10): 1506-1516.
Pitout, J. D. and K. B. Laupland (2008). "Extended-spectrum beta-lactamase-producing Enterobacteriaceae: an emerging public-health concern." Lancet Infect Dis 8(3): 159-166. Prosser, B. L., D. Taylor, B. A. Dix and R. Cleeland (1987). "Method of evaluating effects of antibiotics on bacterial biofilm." Antimicrob Agents Chemother 31(10): 1502-1506.
Schuch, R., D. Nelson and V. A. Fischetti (2002). "A bacteriolytic agent that detects and kills Bacillus anthracis." Nature 418(6900): 884-889.
Silby, M. W., C. Winstanley, S. A. Godfrey, S. B. Levy and R. W. Jackson (2011). "Pseudomonas genomes: diverse and adaptable." FEMS Microbiol Rev 35(4): 652-680.
Smith, E. E., D. G. Buckley, Z. Wu, C. Saenphimmachak, L. R. Hoffman, D. A. D'Argenio, S. I. Miller, B. W. Ramsey, D. P. Speert, S. M. Moskowitz, J. L. Burns, R. Kaul and M. V.
Olson (2006). "Genetic adaptation by Pseudomonas aeruginosa to the airways of cystic fibrosis patients." Proc Natl Acad Sci U S A 103(22): 8487-8492.
Sonnevend, A., A. Ghazawi, R. Hashmey, A. Haidermota, S. Girgis, M. Alfaresi, M. Omar, D. L. Paterson, H. M. Zowawi and T. Pal (2017). "Multihospital Occurrence of Pan-Resistant Klebsiella pneumoniae Sequence Type 147 with an ISEcp1-Directed blaOXA-181 Insertion in the mgrB Gene in the United Arab Emirates." Antimicrob Agents Chemother 61(7).
Spencer, R. C. (1996). "Predominant pathogens found in the European Prevalence of Infection in Intensive Care Study." Eur J Clin Microbiol Infect Dis 15(4): 281-285.
Stock, J. B., B. Rauch and S. Roseman (1977). "Periplasmic space in Salmonella typhimurium and Escherichia coli." J Biol Chem 252(21): 7850-7861.
Thandar, M., R. Lood, B. Y. Winer, D. R. Deutsch, C. W. Euler and V. A. Fischetti (2016). "Novel Engineered Peptides of a Phage Lysin as Effective Antimicrobials against Multidrug-Resistant Acinetobacter baumannii." Antimicrob Agents Chemother 60(5): 2671-2679.
Tsang, K. W. and D. Bilton (2009). "Clinical challenges in managing bronchiectasis." Respirology 14(5): 637-650.
Vaara, M. (1992). "Agents that increase the permeability of the outer membrane." Microbiol Rev 56(3): 395-411.
Veesenmeyer, J. L., A. R. Hauser, T. Lisboa and J. Rello (2009). "Pseudomonas aeruginosa virulence and therapy: evolving translational strategies." Crit Care Med 37(5): 1777-1786.
Walmagh, M., B. Boczkowska, B. Grymonprez, Y. Briers, Z. Drulis-Kawa and R. Lavigne (2013). "Characterization of five novel endolysins from Gram-negative infecting bacteriophages." Appl Microbiol Biotechnol 97(10): 4369-4375.
Walmagh, M., Y. Briers, S. B. dos Santos, J. Azeredo and R. Lavigne (2012). "Characterization of modular bacteriophage endolysins from Myoviridae phages OBP, 201phi2-1 and PVP-SE1." PLoS One 7(5): e36991.
Weissman, B. A., B. J. Mondino, T. H. Pettit and J. D. Hofbauer (1984). "Corneal ulcers associated with extended-wear soft contact lenses." Am J Ophthalmol 97(4): 476-481.
Weterings, V., K. Zhou, J. W. Rossen, D. van Stems, E. Thewessen, J. Kluytmans and J. Veenemans (2015). "An outbreak of colistin-resistant Klebsiella pneumoniae carbapenemase-producing Klebsiella pneumoniae in the Netherlands (July to December 2013), with inter-institutional spread." Eur J Clin Microbiol Infect Dis 34(8): 1647-1655.
Williams, B. J., J. Dehnbostel and T. S. Blackwell (2010). "Pseudomonas aeruginosa: host defence in lung diseases." Respirology 15(7): 1037-1056.
Winstanley, C., S. O'Brien and M. A. Brockhurst (2016). "Pseudomonas aeruginosa Evolutionary Adaptation and Diversification in Cystic Fibrosis Chronic Lung Infections." Trends Microbiol 24(5): 327-337.
Wisplinghoff, H., T. Bischoff, S. M. Tallent, H. Seifert, R. P. Wenzel and M. B. Edmond (2004). "Nosocomial bloodstream infections in US hospitals: analysis of 24,179 cases from a prospective nationwide surveillance study." Clin Infect Dis 39(3): 309-317.
Young, R. (2014). "Phage lysis: three steps, three choices, one outcome." J Microbiol 52(3): 243-258.

## Claims

1. A pharmaceutical composition comprising at least one isolated and/or recombinant lysin polypeptide that is effective to kill bacteria, wherein the lysin polypeptide comprises an amino acid sequence that is 100% identical to an amino acid sequence of a lysin polypeptide of PlyPa91 (SEQ ID NO: 13).

2. The pharmaceutical composition of claim 1, wherein the lysin polypeptide is a recombinant polypeptide.

3. The pharmaceutical composition of claim 2, wherein the lysin polypeptide comprises amino acids encoded by an expression vector used to produce the polypeptide.

4. The pharmaceutical composition of claim 3, wherein the lysin polypeptide is: PlyPa91 (SEQ ID NO: 13).

5. A pharmaceutical composition for use in a method of killing bacteria on or in an individual, the method comprising contacting the bacteria with an effective amount of the pharmaceutical composition of any one of claims 1-4;
wherein the bacteria is from *Enterobacteriaceae;* or is from the genera *Enterobacter, Pseudomonas* or *Klebsiella;* or wherein the bacteria is *A. baumannii, E. coli, S. sonnei, S. flexneri, C. freundii, K. pneumoniae, E. cloacae, E. aerogenenes* or *P. aeruginosa.*

6. The pharmaceutical composition for use of claim 5, wherein the bacteria are in an infection of skin, a wound, and/or mucosa of the individual.

7. An expression vector encoding a recombinant lysin polypeptide as defined in any one of claims 1-4.

8. The expression vector of claim 7, the recombinant polypeptide optionally comprising at least one of: a purification tag, or an amino acid sequence encoded by the expression vector from codons of one or more restriction enzyme recognition sites, or a sequence encoding a protease recognition sequence.

9. Isolated cells comprising an expression vector of claim 7.

10. A method comprising culturing cells of claim 9, separating recombinant lysin polypeptides from the cells, and optionally purifying the recombinant lysin polypeptides, and optionally removing a purification tag using a sequence-dependent protease.

11. A method for determining the presence of bacteria in a sample comprising contacting a sample with a detectably labeled lysin as defined in any one of claims 1-4 and detecting a signal from the detectable label to thereby determine the presence of bacteria in the sample.

## Patentansprüche

1. Pharmazeutische Zusammensetzung, umfassend mindestens ein isoliertes und/oder rekombinantes Lysin-Polypeptid, das zum Abtöten von Bakterien wirksam ist, wobei das Lysin-Polypeptid eine Aminosäuresequenz umfasst, die zu 100 % identisch ist mit einer Aminosäuresequenz eines Lysin-Polypeptids von PlyPa91 (SEQ ID NO: 13).

2. Pharmazeutische Zusammensetzung nach Anspruch 1, wobei das Lysin-Polypeptid ein rekombinantes Polypeptid ist.

3. Pharmazeutische Zusammensetzung nach Anspruch 2, wobei das Lysin-Polypeptid Aminosäuren umfasst, die von einem Expressionsvektor codiert werden, der verwendet wird, um das Polypeptid herzustellen.

4. Pharmazeutische Zusammensetzung nach Anspruch 3, wobei das Lysinpolypeptid Folgendes ist: PlyPa91 (SEQ ID NO: 13).

5. Pharmazeutische Zusammensetzung zur Verwendung in einem Verfahren zum Abtöten von Bakterien auf oder in einem Individuum, wobei das Verfahren Inkontaktbringen der Bakterien mit einer wirksamen Menge der pharmazeutischen Zusammensetzung nach einem der Ansprüche 1-4 umfasst;
wobei die Bakterien aus den *Enterobacteriaceae* stammen; oder aus den Gattungen *Enterobacter, Pseudomonas* oder *Klebsiella* stammen; oder wobei die Bakterien *A*. *baumannii, E. coli, S. sonnei, S. flexneri, C. freundii, K. pneumoniae, E. cloacae, E. aerogenenes* oder *P*. *aeruginosa* sind.

6. Pharmazeutische Zusammensetzung zur Verwendung nach Anspruch 5, wobei die Bakterien in einer Infektion der Haut, einer Wunde und/oder einer Schleimhaut des Individuums vorhanden sind.

7. Expressionsvektor, der ein rekombinantes Lysin-Polypeptid codiert, wie es in einem der Ansprüche 1-4 definiert ist.

8. Expressionsvektor nach Anspruch 7, wobei das rekombinante Polypeptid optional mindestens eines der Folgenden umfasst: ein Reinigungs-Tag oder eine Aminosäuresequenz, die durch den Expressionsvektor von Codonen einer oder mehrerer Restriktionsenzym-Erkennungsstellen codiert wird, oder eine Sequenz, die eine Protease-Erkennungssequenz codiert.

9. Isolierte Zellen, die einen Expressionsvektor nach Anspruch 7 umfassen.

10. Verfahren, Folgendes umfassend: Kultivieren von Zellen nach Anspruch 9, Abtrennen rekombinanter Lysin-Polypeptide von den Zellen und gegebenenfalls Reinigen der rekombinanten Lysin-Polypeptide und gegebenenfalls Entfernen eines Reinigungs-Tags unter Verwendung einer sequenzabhängigen Protease.

11. Verfahren zum Bestimmen des Vorhandenseins von Bakterien in einer Probe, Folgendes umfassend: Inkontaktbringen einer Probe mit einem nachweisbar markierten Lysin, wie in einem der Ansprüche 1-4 definiert, und Nachweisen eines Signals von der nachweisbaren Markierung, um dadurch das Vorhandensein von Bakterien in der Probe zu bestimmen.

## Revendications

1. Composition pharmaceutique comprenant au moins un polypeptide de lysine isolé et/ou recombinant qui est efficace pour tuer les bactéries, dans laquelle le polypeptide de lysine comprend une séquence d'acides aminés qui est identique à 100 % à une séquence d'acides aminés d'un polypeptide de lysine de PlyPa91 (SEQ ID NO : 13).

2. Composition pharmaceutique selon la revendication 1, dans laquelle le polypeptide de lysine est un polypeptide recombinant.

3. Composition pharmaceutique selon la revendication 2, dans laquelle le polypeptide de lysine comprend des acides aminés codés par un vecteur d'expression utilisé pour produire le polypeptide.

4. Composition pharmaceutique selon la revendication 3, dans laquelle le polypeptide de lysine est : PlyPa91 (SEQ ID NO : 13) .

5. Composition pharmaceutique destinée à être utilisée dans un procédé pour tuer des bactéries sur ou dans un individu, le procédé comprenant la mise en contact des bactéries avec une quantité efficace de la composition pharmaceutique selon l'une quelconque des revendications 1 à 4 ;
dans laquelle la bactérie provient *d'Enterobacteriaceae* ; ou appartient aux genres *Enterobacter, Pseudomonas* ou *Klebsiella ;* ou dans laquelle la bactérie est *A*. *baumannii, E. coli, S. sonnei, S. flexneri, C. freundii, K. pneumoniae, E. cloacae, E. aerogenenes* ou *P*. *aeruginosa.*

6. Composition pharmaceutique destinée à être utilisée selon la revendication 5, dans laquelle les bactéries sont présentes dans une infection de la peau, d'une plaie et/ou d'une muqueuse de l'individu.

7. Vecteur d'expression codant pour un polypeptide de lysine recombinant tel que défini dans l'une quelconque des revendications 1 à 4.

8. Vecteur d'expression selon la revendication 7, le polypeptide recombinant comprenant éventuellement au moins l'une parmi : un marqueur de purification, ou une séquence d'acides aminés codée par le vecteur d'expression à partir de codons d'un ou plusieurs sites de reconnaissance d'enzymes de restriction, ou une séquence codant pour une séquence de reconnaissance de protéase.

9. Cellules isolées comprenant un vecteur d'expression selon la revendication 7.

10. Procédé comprenant la culture de cellules selon la revendication 9, la séparation de polypeptides de lysine recombinants des cellules, et, éventuellement, la purification des polypeptides de lysine recombinants, et éventuellement l'élimination d'un marqueur de purification en utilisant une protéase dépendante de la séquence.

11. Procédé de détermination de la présence de bactéries dans un échantillon comprenant la mise en contact d'un échantillon avec une lysine marquée de manière détectable telle que définie dans l'une quelconque des revendications 1 à 4 et la détection d'un signal provenant du marqueur détectable pour ainsi déterminer la présence de bactéries dans l'échantillon.
